(19) Europäisches Patentamt
European Patent Office
Office européen des brevets

(11) **EP 4 223 273 B1**

(12) **EUROPEAN PATENT SPECIFICATION**

(45) Date of publication and mention
of the grant of the patent:
**28.01.2026 Bulletin 2026/05**

(51) International Patent Classification (IPC):
***A61K 8/02*** *(2006.01)*     ***A61K 8/73*** *(2006.01)*

(21) Application number: **23154388.5**

(52) Cooperative Patent Classification (CPC):
**A61K 8/731; A61K 8/0241; A61K 8/361;
A61K 8/44; A61K 8/84; A61K 8/922; A61Q 1/00;
A61Q 19/00;** A61K 2800/412; A61K 2800/612;
A61K 2800/614; A61K 2800/654

(22) Date of filing: **01.02.2023**

(54) **CELLULOSIC PARTICLE**

ZELLULOSEPARTIKEL

PARTICULE CELLULOSIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **08.02.2022 JP 2022017985
29.07.2022 JP 2022122215**

(43) Date of publication of application:
**09.08.2023 Bulletin 2023/32**

(73) Proprietor: **FUJIFILM Business Innovation Corp.
Minato-ku
Tokyo (JP)**

(72) Inventors:
• **YAO, Kenji
Minamiashigara-shi, Kanagawa (JP)**
• **OKI, Masahiro
Minamiashigara-shi, Kanagawa (JP)**
• **YOSHIKAWA, Hideaki
Minamiashigara-shi, Kanagawa (JP)**
• **YOSHIDA, Kazusei
Minamiashigara-shi, Kanagawa (JP)**
• **NAITO, Ayu
Minamiashigara-shi, Kanagawa (JP)**
• **IWADATE, Yuko
Minamiashigara-shi, Kanagawa (JP)**
• **ISHIZUKA, Takahiro
Minamiashigara-shi, Kanagawa (JP)**
• **TAGUCHI, Tetsuya
Minamiashigara-shi, Kanagawa (JP)**
• **HAMANO, Hirokazu
Minamiashigara-shi, Kanagawa (JP)**
• **KASHIWAGI, Satomi
Minamiashigara-shi, Kanagawa (JP)**

(74) Representative: **Kurig, Thomas
Becker Kurig & Partner
Patentanwälte mbB
Bavariastraße 7
80336 München (DE)**

(56) References cited:
**EP-A- 0 273 890        EP-A- 0 421 581
WO-A1-2016/098910     WO-A1-2016/174503
WO-A2-2017/104587**

## Description

Background

(i) Technical Field

The present disclosure relates to a cellulosic particle.

(ii) Related Art

In Japanese Patent No. 6872068, "resin beads formed of a resin containing cellulose as a main component, wherein the particle size at a cumulative percentage of 50% in terms of volume is 50 $\mu$m or less, the sphericity is 0.7-1.0, the surface smoothness is 70-100%, the solidity is 50-100%, the five-day biodegradability measured according to JIS K6950:2000 (ISO 14851:1999) is 20% or greater, and the content of cellulose in the resin is 90-100 mass%." are proposed.

In Japanese Patent No. 6855631, "a powdered cellulose which has a mean particle diameter of 5 to 150 $\mu$m, and an in-water sonication residual ratio of 20 to 60%, the in-water sonication residual ratio (%) represented by [particle diameter at 50% cumulative total volume by wet method measurement (with ultrasound irradiation) / particle diameter at 50% cumulative total volume by wet method measurement (without ultrasound irradiation)] $\times$ 100." is proposed. WO 2017/104587 A2 discloses a composite particle including (a) a core particle, wherein the (a) core particle is covered with at least one first coating layer comprising (b) a solid UV filter, and the first coating layer is covered with at least one second coating layer comprising (c) a hydrophobic block copolymer, as well as a cosmetic composition including the composite particle.

WO 2016/174503 A1 discloses a composition for cosmetic raw material containing microcapsule containing a encapsulated material comprising a core and a layered coating surrounding the core, and the encapsulated material being a particle having a high wet point and being optionally porous, and being only released from the microcapsule when the composition is applied onto a keratin material, such as keratin fibers or skin.

WO 2016/098910 A1 discloses a composite particle comprising: a hydrophilic core particle; and a plurality of hydrophobic particles, wherein the surface of the hydrophilic core particle is discontinuously covered by the hydrophobic particles.

EP 0 273 890 A1 discloses a dosage form for oral administration of a pharmaceutically active substance with a encapsulated or embedded pharmaceutically active substance in a pharmaceutically acceptable non-aqueous liquid.

EP 0 421 581 A1 discloses a spray dried spheroidal microcapsule under about 150 microns in diameter which comprises (a) a medicament present in an amount from about 1% to about 90%, by weight of the microcapsule composition, (b) a film forming polymer present in an amount from about 8% to about 90%, by weight of the microcapsule composition, (c) a plasticizing agent in an amount from about 5% to about 30%, by weight of the film forming polymer.

Summary

The present invention is provided in the appended claims. The following disclosure serves a better understanding of the present invention. Accordingly, it is an object of the present disclosure to provide a cellulosic particle that is highly biodegradable and exhibits little change in texture over time compared with cellulosic particles containing cellulose as their base constituent and whose 5-day or 60-day percentage biodegradation measured as per JIS K6950:2000 exceeds 20% or is lower than 60%, respectively.

Detailed Description

Exemplary embodiments of the present disclosure will now be described. The following description and the Examples are for illustrating exemplary embodiments and do not limit the scope of aspects of the present disclosure.

In a series of numerical ranges presented herein, the upper or lower limit of a numerical range may be substituted with that of another in the same series. The upper or lower limit of a numerical range, furthermore, may be substituted with a value indicated in the Examples section.

A constituent may be a combination of multiple substances.

If a composition contains a combination of multiple substances as one of its constituents, the amount of the constituent represents the total amount of the substances in the composition unless stated otherwise.

Cellulosic Particles

Cellulosic particles according to an exemplary embodiment contain cellulose as their base constituent, and the 5-day and 60-day percentage biodegradations of the cellulosic particles measured as per JIS K6950:2000 are lower than 20% and 60% or higher, respectively.

Configured as described above, the cellulosic particles according to this exemplary embodiment are highly biodegradable and exhibit little change in texture over time. Possible reasons are as follows.

Due to the issue of marine debris, there is a need for biodegradable resin particles. In particular, cellulosic particles containing cellulose as their base constituent have been used in various practical applications, such as cosmetics, by virtue of their rapid biodegradation in all of compost, activated sludge, and seawater environments.

Known cellulosic particles, however, are decomposed too rapidly in the initial stage of biodegradation; the associated decrease in the mechanical strength of the surface of the particles causes chipping and other defects, resulting in the surface texture (feel of the surface when touched, such as smoothness, moist sensation, and softness) of the particles deteriorating over time even under normal use conditions.

Usually, the biodegradation of cellulosic particles starts at the surface of the particles (the point of contact with the degrading medium). Cellulosic particles with high initial biodegradability, therefore, experience a decrease in the molecular weight of cellulose specifically on their very surface. The resulting decrease in the strength of the surface makes the particles more prone to minor chipping and deformation. Limiting the initial biodegradability of cellulosic particles helps control the chipping and deformation of the surface of the particles that occur over time, and this helps reduce changes in the texture of the particles over time.

More specifically, making the 5-day percentage biodegradation of cellulosic particles measured as per JIS K6950:2000 lower than 20% leads to reduced initial biodegradability of the particles. This helps reduce changes in the texture of the particles over time by helping control the chipping and deformation of the surface of the particles over time.

Making the 60-day percentage biodegradation of the cellulosic particles measured as per JIS K6950:2000 equal to or higher than 60%, furthermore, ensures that the particles remain highly biodegradable.

For these reasons, presumably, the cellulosic particles according to this exemplary embodiment, configured as described above, are highly biodegradable and exhibit little change in texture over time.

Specifically, the cellulosic particles according to this exemplary embodiment exhibit little change in their feel when touched, such as smoothness, moist sensation, and softness, by virtue of the small changes in their texture over time.

The details of the cellulosic particles according to this exemplary embodiment will now be described.

Cellulose

The cellulosic particles according to this exemplary embodiment contain cellulose as their base constituent.

In this context, the term containing cellulose as a base constituent (or "cellulose-based") means the cellulose content of the cellulosic particles is 90% by mass or more.

If the cellulosic particles have a coating layer as described later herein, containing cellulose as a base constituent (or cellulose-based) means the cellulose content of the core particle is 90% by mass or more.

The number-average molecular weight of the cellulose may be 37000 or more, preferably 45000 or more.

There is no particular upper limit, but for example, the number-average molecular weight of the cellulose may be 100000 or less.

Making the number-average molecular weight of the cellulose 37000 or more makes more certain that the cellulosic particles are highly biodegradable and exhibit little change in texture over time. Possible reasons are as follows.

If the number-average molecular weight of the cellulose is too low, the initial rate of biodegradation tends to be out of control because of too rapid biodegradation. Making the molecular weight 37000 or more helps reduce changes in texture over time by helping control the chipping and deformation of the surface of the particles. If the number-average molecular weight is too low, furthermore, the disintegration of the particles is somewhat nonuniform because of too rapid initial biodegradation; the resulting variations in size between particles will lead to a slow overall rate of biodegradation. Making the molecular weight 37000 or more helps ensure uniform disintegration, and therefore superior biodegradability, of the particles.

For these reasons, presumably, it is more certain that the cellulosic particles are highly biodegradable and exhibit little change in texture over time.

The number-average molecular weight of the cellulose is measured by gel permeation chromatography (differential refractometer, Optilab T-rEX, Wyatt Technology; multiangle light scattering detector, DAWN HELEOS II, Wyatt Technology; columns, one TSKgel $\alpha$-M and one $\alpha$-3000, Tosoh) with dimethylacetamide eluent (containing 0.1 M lithium chloride).

Extra Constituents

The cellulosic particles according to this exemplary embodiment may contain extra constituents. If the cellulosic particles have a coating layer as described later herein, the extra constituents are contained in the core particle, covered with the coating layer.

Examples of extra constituents include plasticizers, flame retardants, compatibilizers, release agents, light stabilizers, weathering agents, coloring agents, pigments, modifiers, antidripping agents, antistatic agents, anti-hydrolysis agents, fillers, reinforcing agents (glass fiber, carbon fiber, talc, clay, mica, glass flakes, milled glass, glass beads, crystalline silica, alumina, silicon nitride, aluminum nitride, boron nitride, etc.), acid acceptors for preventing acetic acid release (oxides, such as magnesium oxide and aluminum oxide; metal hydroxides, such as magnesium hydroxide, calcium hydroxide, aluminum hydroxide, and hydrotalcite; calcium carbonate; talc; etc.), and reactive trapping agents (e.g., epoxy compounds, acid anhydride compounds, carbodiimides, etc.).

The amount of each extra constituent may be 0% by mass or more and 5% by mass or less of the cellulosic particles (or core particles) as a whole. In this context, "0% by mass" means the cellulosic particles (or core particles) are free of that extra constituent.

Percentage Biodegradations

The 5-day percentage biodegradation of the cellulosic particles according to this exemplary embodiment measured as per JIS K6950:2000 is lower than 20%. For the reduction of changes in texture over time, the 5-day percentage biodegradation may be 15% or lower, preferably 10% or lower.

The 5-day percentage biodegradation may ideally be 0%, but it is difficult to completely eliminate initial biodegradability because the material used is biodegradable by nature; therefore, the 5-day percentage biodegradation is, for example, 5% or higher.

The 60-day percentage biodegradation of the cellulosic particles according to this exemplary embodiment measured as per JIS K6950:2000 is 60% or higher. For high biodegradability, the 60-day percentage biodegradation may be 65% or higher, preferably 70% or higher.

Higher 60-day percentage biodegradations may be better, but usually, this percentage cannot be 100%, for example because of limited precision in measuring the BOD, or precision in the detection of oxygen, and the influence of oxygen consumption by microorganisms not involving the decomposition of the sample; therefore, the 60-day percentage biodegradation is, for example, 95% or lower.

These percentage biodegradations are measured as per JIS K6950:2000. JIS K6950:2000 corresponds to ISO 14851:1999.

Specifically, the percentage biodegradations are calculated from the oxygen demands of the cellulosic particles of interest (hereinafter, the test substance) and a reference substance according to the equation below.

$$\text{Biodegradation} (\%) = (A-B)/C \times 100$$

A (mg): Biochemical oxygen demand of the test substance
B (mg): Mean biochemical oxygen demand of the control substance
C (mg): Theoretical maximum amount of oxygen required to oxidize the test substance

The oxygen demands, furthermore, are measured using a closed-system oxygen consumption meter under the following conditions.

Inoculum: Activated sludge in an aerobic reactor at a sewage treatment plant basically for the treatment of domestic liquid waste
Control substance: Microcrystalline cellulose
Test substance concentration: 100 mg/L
Control substance concentration: 100 mg/L
Inoculum concentration: 150 mg/L
Test solution volume: 300 mL
Testing temperature: 25°C±1°C
Duration of incubation: 30 days

Coated Cellulosic Particles

The cellulosic particles according to this exemplary embodiment may be cellulosic particles having a cellulose-based core particle (hereinafter also referred to as a cellulosic core particle) and a coating layer covering the core particle and containing at least one selected from the group consisting of a polyamine compound, an arginine compound, a wax, a linear-chain fatty acid, a linear-chain fatty acid metallic salt (metallic salt of a linear-chain fatty acid), a hydroxy fatty acid, and an amino acid compound (hereinafter also referred to as "coated cellulosic particles").

This configuration makes more certain that the cellulosic particles according to this exemplary embodiment are highly biodegradable and exhibit little change in texture over time. Possible reasons are as follows.

A polyamine compound adheres to the surface of the cellulose with its affinity for hydroxyl groups. The adhesion of the polyamine compound, therefore, helps control initial biodegradation of the surface of the cellulosic particles, and this helps reduce changes in texture over time. The polyamine compound, furthermore, does not cover the surface completely but leaves portions of the surface exposed. Since microorganisms can pass through the spaces left on the surface, the superior biodegradability of the cellulose will be reflected in that of the particles after time.

An arginine compound covers part of the cellulosic core particle through ionic bonding between its terminal carboxylic acid and hydroxyl groups on the surface of the cellulosic core particle. It appears that a seamless array of exposed portions and portions covered with the arginine compound is formed on the cellulosic core particle, and the resulting delicate irregularities and unevenness in hygroscopic capacity helps reduce changes in texture over time. Although initial biodegradation is limited because the covered portions are less biodegradable than the cellulosic core particle itself, the entire particles will be biodegrade after time because the arginine compound is also biodegradable.

A wax, a linear-chain fatty acid, and a linear-chain fatty acid metallic salt, highly water-repellent in themselves, inhibit the hydrolysis of the cellulose by making the particles more hydrophobic, and the uniform progress of the biodegradation of the particles without surface chipping in the initial stage of biodegradation enabled by this helps reduce changes in texture over time. These compounds also help achieve superior biodegradability; they leave exposed portions on the surface of the core particle with their tendency to partial aggregation, providing spaces for microorganisms to penetrate through.

A hydroxy fatty acid adheres to the surface of the cellulosic particles through weak hydrogen bonding between its hydroxyl group and hydroxyl groups of the cellulosic particles. The fatty acid moiety of the adhering hydroxy fatty acid, facing outwards from the particle, inhibit initial hydrolysis of the cellulose by improving the hydrophobicity of the particle, and the inhibited initial hydrolysis of the cellulose helps reduce changes in texture over time by preventing surface chipping. The hydrocarbon moiety of the fatty acid, furthermore, is spaced apart from the cellulose because of its low affinity for cellulose; microorganisms can penetrate into the cellulosic particles through the spaces, and the uniform progress of biodegradation enabled by this helps achieve superior biodegradability.

An amino acid compound has a strong tendency to form flat-shaped crystals after coating; these crystals help limit initial contact between microorganisms and the cellulose with their large specific surface area, and the resulting delayed biodegradation leads to reduced changes in texture over time. The crystals, furthermore, are formed with spaces therebetween, through which microorganisms can penetrate slowly; the resultant uniform progress of biodegradation helps achieve superior biodegradability.

For these reasons, presumably, it is more certain that the cellulosic particles are highly biodegradable and exhibit little change in texture over time.

The cellulosic particles according to the present invention include a cellulose-based core particle produced by saponifying a cellulose acylate to have more hydroxyl groups on its surface than inside. This helps cover the core particle with the coating layer with a high coverage.

Cosmetics made with the coated cellulosic particles, furthermore, produce superior skin feelings (smoothness, moist sensation, and softness) even at high or low temperatures. Possible reasons are as follows.

The biodegradation of coated cellulosic particles is initiated by one or both of the following two events.

(1) Degrading microorganisms pass through the coating layer and biodegrade the cellulosic core particle, which rapidly biodegrades by nature.
(2) Microorganisms decompose the coating layer itself.

If the 5-day percentage biodegradation measured as per JIS K6950 (ISO 14581:1999) is as high as 20% or higher, what drives the process is event (1), the decomposition of the rapidly biodegradable cellulosic core particle. Under normal temperature conditions, biodegradation would not affect the feelings the particles produce on the skin in cosmetic use, because the structure of the coating layer would remain. The surface of the cellulosic core particle, however, would be decomposed, and the coating layer would lose a ground for it to lie on; part of it would no longer be bound to the surface of the cellulosic core particle.

At low ambient temperatures of 0°C or below, the coating layer becomes brittle because molecular motions in its structure are frozen. Even in this situation, the structure of the coating layer is not broken as long as the coating layer is sticking to the cellulosic core particle, because the cellulosic core particle is strong even at low temperatures.

Since the 5-day percentage biodegradation of the cellulosic particles having a surface layer measured as per JIS K6950 (ISO 14581:1999) is as high as 20% or higher, however, part of the structure of the surface layer is not bound to the cellulosic particles; the structure of the surface layer breaks, starting from the detached portions.

In this way, low temperatures of 0°C or below affect the feelings the cellulosic particles having a surface layer produce on the skin in cosmetic use (specifically, smoothness, moist sensation, softness, etc.), if the 5-day percentage degradation of the particles measured as per JIS K6950 (ISO 14581:1999) is as high as 20% or higher.

At high ambient temperatures of 60°C or above, the structure of the coating layer deforms easily. If the coating layer is bound uniformly to the cellulosic core particle, the impact of the deformation is minimal; if the 5-day percentage degradation measured as per JIS K6950 (ISO 14581:1999) is as high as 20% or higher, however, the deformation affects the feelings the coated cellulosic particles produce on the skin in cosmetic use (specifically, smoothness, moist sensation, softness, etc.) because part of the structure of the coating layer is not bound to the cellulosic core particle.

If the 60-day percentage biodegradation measured as per JIS K6950 (ISO 14581:1999) is lower than 60%, the cellulosic core particle is totally inaccessible by microorganisms, for example in a form like the surface of the cellulosic particles is densely covered with a slowly biodegradable compound, and if such cellulosic particles are placed at low temperatures of 0°C or below or high temperatures of 60°C or above, their surface layer cracks due to the difference in linear expansion between it and the cellulosic core particle, making the surface very rough. This affects the feelings the cellulosic particles produce on the skin in cosmetic use (specifically, smoothness, moist sensation, softness, etc.).

For these reasons, presumably, cosmetics made with the coated cellulosic particles produce superior skin feelings (smoothness, moist sensation, and softness) even at high or low temperatures.

Core Particle

The core particle is a cellulose-based particle.

The cellulose contained in the core particle has the same definition as the cellulose previously described herein; possible and preferred ranges of parameters are also the same as in the foregoing.

Coating Layer

The coating layer contains at least one selected from the group consisting of a polyamine compound, a wax, an arginine compound, a linear-chain fatty acid, a linear-chain fatty acid metallic salt (metallic salt of a linear-chain fatty acid), a hydroxy fatty acid, and an amino acid compound.

- Polyamine Compound

"Polyamine compound" is a generic term for, in accordance with the present invention, aliphatic hydrocarbons having two or more primary amino groups.

Examples of polyamine compounds, in accordance with the present invention, are a polyalkyleneimine, polyallylamine, polyvinylamine, and polylysine.

For improved biodegradability, the polyalkyleneimine may be a polyalkyleneimine including a repeat unit having an alkylene group with one or more and six or fewer carbon atoms (C1 to C6; preferably C1 to C4, more preferably C1 or C2), preferably polyethyleneimine.

Examples of polyallylamines include homopolymers or copolymers of allylamine, allylamine amidosulfate, diallylamine, dimethylallylamine, etc.

Examples of polyvinylamines include products of alkali hydrolysis of poly(N-vinylformamide); a specific example is Mitsubishi Chemical's "PVAM-0595B."

The polylysine may be an extract from a natural source, may be a substance produced by a transformed microorganism, or may be a product of chemical synthesis.

The polyamine compound may be at least one selected from the group consisting of polyethyleneimine and polylysine.

Using at least one selected from the group consisting of polyethyleneimine and polylysine as polyamine compound(s) makes more certain that the cellulosic particles are highly biodegradable and exhibit little change in texture over time. Possible reasons are as follows.

Polyethyleneimine and polylysine are able to adhere firmly to the cellulosic particles by virtue of their high cation density and functional groups that react with the hydroxyl groups in the cellulose. Their hydrocarbon chain, at the same time, takes up an appropriate relative area, so if they adhere to the surface of the cellulosic particles, the hydrocarbon chains tend to be exposed on the surface; the resulting increase in the hydrophobicity of the particles prevents surface defects by slowing down initial hydrolysis and biodegradation of the cellulose, and the uniform progress of biodegradation enabled by this reduces changes in texture over time. Polyethyleneimine and polylysine, furthermore, are not dense but relatively loose in terms of structure, which means that they provide spaces for microorganisms to penetrate through; the superior biodegradability of the cellulose, therefore, is reflected in that of the particles.

For these reasons, presumably, it is more certain that the cellulosic particles are highly biodegradable and exhibit little change in texture over time.

The polyamine compound content may be 0.2% by mass or more and 2% by mass or less of the cellulosic particles as a whole.

- Arginine Compound

Arginine compounds, in accordance with the present invention, are compounds having the structure of 2-amino-5-guanidinopentanoic acid (2-amino-5-guanidinovaleric acid).

Examples of arginine compounds, in accordance with the present invention, are L-arginine, D-arginine, 2-amino-3-methyl-5-guanidinopentanoic acid, 2-amino-3-ethyl-5-guanidinopentanoic acid, and 2-amino-3,3-dimethyl-5-guanidinopentanoic acid.

The arginine compound content may be 0.1% by mass or more and 5% by mass or less of the cellulosic particles as a whole.

- Wax

Examples of waxes, in accordance with the present invention, include fatty acid-containing vegetable oils, hydrocarbon waxes, and diesters.

Examples of fatty acid-containing vegetable oils, in accordance with the present invention, are castor oil, paulownia oil, linseed oil, shortening, corn oil, soybean oil, sesame oil, rapeseed oil, sunflower oil, rice bran oil, camellia oil, coconut oil, palm oil, walnut oil, olive oil, peanut oil, almond oil, jojoba oil, cocoa butter, shea butter, neem oil, safflower oil, Japan wax, candelilla wax, rice bran wax, carnauba wax, and *Rosa damascena* flower wax.

Examples of hydrocarbon waxes are petroleum waxes (paraffin wax, microcrystalline wax, petrolatum wax, etc.) and synthetic hydrocarbon waxes (polyethylene wax, polypropylene wax, polybutene wax, Fischer-Tropsch wax, etc.).

Examples of diesters are diesters of dibasic acids, such as malic acid, glutaric acid, adipic acid, azelaic acid, sebacic acid, and dodecanedioic acid, and C10 to C25 alcohols.

The wax may be carnauba wax.

Using carnauba wax as a wax makes more certain that the cellulosic particles are highly biodegradable and exhibit little change in texture over time. Possible reasons are as follows.

Carnauba wax is highly effective in reducing changes in texture over time because constituents having a water-repellent structure abundant therein, such as free fatty acids and hydrocarbons, help prevent initial hydrolysis of the cellulosic particles and enable uniform progress of biodegradation without surface chipping; carnauba wax, furthermore, helps achieve superior biodegradability if enough time is allowed, because it adheres to the cellulosic particles through weak hydrogen bonding between free alcohols it contains and hydroxyl groups of the cellulosic particles, but with spaces at the interface through which microorganisms can penetrate by virtue of relatively weak adhesive strength.

For these reasons, presumably, it is more certain that the cellulosic particles are highly biodegradable and exhibit little change in texture over time.

The wax content may be 0.1% by mass or more and 2% by mass or less, preferably 0.2% by mass or more and 1% by mass or less, of the cellulosic particles as a whole.

- Linear-Chain Fatty Acid

Linear-chain fatty acids are saturated or unsaturated fatty acids in a linear-chain structure. The linear-chain fatty acid may be a mixture of saturated and unsaturated fatty acids.

For improved biodegradability and smaller changes in texture over time, in accordance with the present invention, the linear-chain fatty acid are a C14 to C22 linear-chain fatty acid. Specific examples of C14 to C22 linear-chain fatty acids-, in accordance with the present invention, are behenic acid, arachidic acid, and palmitic acid.

The reason why using a linear-chain fatty acid in the coating layer helps reduce changes in the texture of the particles over time and achieve superior biodegradability appears to be as follows. The terminal carboxylic acid is able to adhere to the surface of the cellulosic particles by forming covalent bonds with, or by virtue of its ionic affinity for, hydroxyl groups of the cellulose. On the surface, linear-chain hydrocarbon groups are exposed and inhibit the hydrolysis of the cellulose by making the particles more hydrophobic, and the uniform progress of the biodegradation of the particles without surface chipping in the initial stage of biodegradation enabled by this helps reduce changes in texture over time. This compound, furthermore, helps achieve superior biodegradability because it creates a porous portion on the surface because of its tendency to partial aggregation, and microorganisms can penetrate into the particles through spaces in this portion.

If the number of carbon atoms in the linear-chain fatty acid is 14 or more, the effectiveness of the fatty acid in preventing changes in texture over time and the biodegradability of the particles are both sufficiently high because in that case the partial aggregation of the fatty acid is sufficiently strong. If the number of carbon atoms is 22 or fewer, however, the linear-chain fatty acid tends to be insufficiently effective in preventing changes in texture over time; in that case, the weakening of its adhesion to the surface of the cellulosic particles is limited because the aggregation of the fatty acid in unlikely to be strong.

The linear-chain fatty acid content may be 2% by mass or more and 15% by mass or less, preferably 5% by mass or more and 10% by mass or less, of the cellulosic particles as a whole.

- Linear-Chain Fatty Acid Metallic Salt

A linear-chain fatty acid metallic salt is, in accordance with the present invention, a metallic salt of a linear-chain saturated or unsaturated fatty acid. The linear-chain fatty acid metallic salt may be a mixture of metallic salts of saturated and unsaturated fatty acids.

Examples of fatty acid metallic salts, in accordance with the present invention, are metallic salts of C10 to C25 (preferably C12 to C22) fatty acids. Examples of metallic salts of C10 to C25 fatty acids include metallic salts of stearic acid, palmitic acid, lauric acid, oleic acid, linoleic acid, and ricinoleic acid.

An example of a metal in a linear-chain fatty acid metallic salt is a divalent metal.

Examples of metals in linear-chain fatty acid metallic salts, in accordance with the present invention, are magnesium, calcium, aluminum, barium, and zinc.

The linear-chain fatty acid metallic salt content may be 2% by mass or more and 15% by mass or less, preferably 5% by mass or more and 10% by mass or less, of the cellulosic particles as a whole.

- Hydroxy Fatty Acid

For improved biodegradability and smaller changes in texture over time, the hydroxy fatty acid may be a C12 to C20 hydroxy fatty acid.

Examples of C12 to C20 hydroxy fatty acids include hydroxystearic acid, hydroxypalmitic acid, hydroxylauric acid, hydroxymyristic acid, and hydrogenated castor oil fatty acids.

The reason why using a hydroxy fatty acid in the coating layer helps prevent changes in the texture of the particles over time and achieve superior biodegradability appears to be as follows. The hydroxy fatty acid adheres to the surface of the cellulosic particles through weak hydrogen bonding between its hydroxyl group and hydroxyl groups of the cellulosic particles. The fatty acid moiety of the adhering hydroxy fatty acid, facing outwards from the particle, inhibit initial hydrolysis of the cellulose by improving hydrophobicity, and the inhibited initial hydrolysis of the cellulose helps reduce changes in texture over time by preventing surface chipping. The hydrocarbon moiety of the fatty acid, furthermore, is spaced apart from the cellulose because of its low affinity for cellulose; microorganisms can penetrate into the cellulosic particles through the spaces, and the uniform progress of biodegradation enabled by this helps achieve superior biodegradability.

If the number of carbon atoms in the hydroxy fatty acid is 12 or more, the effectiveness of the fatty acid in reducing changes in texture over time tends to be improved because in that case it is unlikely that the repulsion between molecules of the fatty acid is weak, and, therefore, hydrophobicity is improved. In the opposite case, or if the number of carbon atoms is 20 or fewer, biodegradability tends to be improved because in that case it is unlikely that long chains of the fatty acid become entangled together, and, therefore, the associated blockage of pathways for microorganisms to enter through is reduced.

The hydroxy fatty acid content may be 1% by mass or more and 10% by mass or less, preferably 3% by mass or more and 10% by mass or less, of the cellulosic particles as a whole.

- Amino Acid Compound

"Amino acid compounds" , in accordance with the present invention, refers to amino acids and amino acid derivatives.

Examples of amino acid compounds, in accordance with the present invention, are lauryl leucine, lauryl arginine, and myristyl leucine.

The reason why using an amino acid compound in the coating layer helps prevent changes in the texture of the particles over time and achieve superior biodegradability appears to be as follows. An amino acid compound has a strong tendency to form flat-shaped crystals after coating; these crystals help limit initial contact between microorganisms and the cellulose with their large specific surface area, and the resulting delayed biodegradation leads to reduced changes in texture over time. The crystals, furthermore, are formed with spaces therebetween, through which microorganisms can penetrate slowly; the resultant uniform progress of biodegradation helps achieve superior biodegradability.

The amino acid compound content may be 2% by mass or more and 10% by mass or less of the cellulosic particles as a whole.

- Layer Structure of the Coating Layer

The coating layer has a first coating layer covering the core particle and containing at least one selected from the group consisting of a polyamine compound, polyvinyl alcohol, polyvinylpyrrolidone, and an arginine compound and a second coating layer covering the first coating layer and containing at least one selected from the group consisting of a wax, a linear-chain fatty acid, a linear-chain fatty acid metallic salt, a hydroxy fatty acid, and an amino acid compound.

In particular, the coating layer has a first coating layer covering the core particle and containing at least one selected from the group consisting of a polyamine compound, an arginine compound, a linear-chain fatty acid, a hydroxy fatty acid, and an amino acid compound and a second coating layer covering the first coating layer and containing at least one selected from the group consisting of a wax, a linear-chain fatty acid, a linear-chain fatty acid metallic salt, a hydroxy fatty acid, and an amino acid compound. The first and second coating layers, however, contain different compound(s).

The presence of such first and second coating layers in the coating layer makes more certain that the cellulosic particles are highly biodegradable and exhibit little change in texture over time. Possible reasons are as follows.

A wax is highly water-repellent and produces strong repulsive forces, but its tendency to self-aggregate often results in the formation of large defects in the coating layer. If these defects are too large, the effectiveness of the coating layer in inhibiting the hydrolysis of the cellulose can be affected, causing chipping of the surface of the particles that can make the reduction of changes in texture over time less significant. Coating the surface with a certain amount of the wax helps prevent the formation of defects, but too much wax, in turn, tends to affect biodegradability. A linear-chain fatty acid and a fatty acid metallic salt tend to be highly crystallizable depending on factors such as ambient temperature, and once crystallized, they can lose some of their adhesiveness to the cellulosic core particle; coating the surface with a certain amount of the fatty acid or metallic salt helps prevent this, but too much fatty acid or metallic salt, in turn, tends to affect biodegradability.

A polyamine compound, hydroxy fatty acid, amino acid compound, or arginine compound only produces weaker repulsive forces than a wax, but its high adhesiveness to the cellulosic particles helps reduce defects in the coating layer. A polyamine compound, a linear-chain fatty acid, a hydroxy fatty acid, and an amino acid compound, furthermore, adhere firmly to a wax, and vice versa; using such a compound, therefore, discourages the formation of coating defects that occur when a wax is used.

For these reasons, the presence of first and second coating layers as described above in the coating layer makes more certain that the cellulosic particles exhibit little change in texture over time. Even if it is a bilayer one, the coating layer still has spaces in it for microorganisms to slowly penetrate through; the biodegradation process, therefore, proceeds more uniformly, and this helps achieve superior biodegradability.

For these reasons, presumably, it is more certain that the cellulosic particles are highly biodegradable and exhibit little change in texture over time.

Cosmetics made with the cellulosic particles whose coating layer has such first and second coating layers, furthermore, produce superior skin feelings (smoothness, moist sensation, and softness) even at high or low temperatures. Possible reasons are as follows.

The second coating compound(s) is effective for smoothness and softness by virtue of its high hydrophobicity and water repellency. As for moist sensation, the compound(s) tends to be somewhat detrimental to the hygroscopicity and water retention of the cellulosic core particle. The first coating layer is able to tie the cellulosic core particle and the second coating layer firmly together by virtue of its compatibility with and ability to bind with both the cellulosic core particle and the first coating layer. The resulting strong influence of the hygroscopicity and water retention of the cellulosic core particle on the second coating layer helps improve moist sensation, too.

In particular, the coating layer may have a first coating layer covering the core particle and containing at least one selected from the group consisting of a polyamine compound and an arginine compound and a second coating layer covering the first coating layer and containing at least one selected from the group consisting of a linear-chain fatty acid, a linear-chain fatty acid metallic salt, and an amino acid compound so that cosmetics made with the cellulosic particles will produce superior skin feelings (smoothness, moist sensation, and softness) even at high or low temperatures. Possible reasons are as follows.

A linear-chain fatty acid and a linear-chain fatty acid metallic salt, having a fatty acid moiety that provides superior hydrophobicity and water repellency, tend to undergo ionic bonding with the cellulosic core particle with their carboxylic acid or carboxylic acid metallic salt moiety, and the resulting concentration of the linear-chain fatty acid moiety in the position closer to the surface of the cellulosic core particle leads to improved smoothness and softness. An amino acid compound has only a short aliphatic length, but its terminal amino acid binds with the first coating layer very firmly; the short aliphatic, therefore, gathers on the surface and improves smoothness and softness. Using a polyamine compound or arginine compound in the first coating layer, furthermore, helps make the cellulosic particles superior in all of skin feelings, smoothness, moist sensation, and softness because these compounds have a particularly powerful effect in keeping the second and first coating layers close to each other yet are harmless to the moist sensation of the cellulosic core particle; a polyamine compound has an amino group at both ends, and one of them binds firmly with hydroxyl groups on the cellulosic core particle with the other binding firmly with carboxylic or amino acid(s) on the second coating layer; an arginine compound has a terminal amino acid that binds with hydroxyl groups of the cellulose and a guanidine structure that binds with carboxylic or amino acid(s) on the second coating layer.

- Polyvalent Metal Salt

The second coating layer may contain a polyvalent metal salt.

The presence of a polyvalent metal salt in the second coating layer makes more certain that the cellulosic particles are highly biodegradable and exhibit little change in texture over time. A possible reason is as follows.

A wax contained in the second layer adheres to the layer beneath it only weakly. The resulting coating, therefore, tends to easily have defects as a result of self-aggregation of the wax. If a polyvalent metal is contained in the second coating layer together with the wax, the polyvalent metal salt spreads uniformly throughout the wax, providing starting points for the wax to aggregate uniformly and extensively; this limits the formation of defects in the coating caused by the self-aggregation of the wax and encourages the adhesion of the second coating layer.

For this reason, presumably, it is more certain that the cellulosic particles are highly biodegradable and exhibit little change in texture over time.

Polyvalent metal salts are compounds formed by a divalent or higher-valency metal ion and an anion.

Examples of divalent or higher-valency metal ions as a component of a polyvalent metal salt include the ions of calcium, magnesium, copper, nickel, zinc, barium, aluminum, titanium, strontium, chromium, cobalt, iron, etc.

Examples of anions as a component of a polyvalent metal salt include inorganic or organic ions. Examples of inorganic ions include the chloride, bromide, iodide, nitrate, sulfate, and hydroxide ions. Examples of organic ions include organic acid ions, such as the carboxylate ion.

Examples of polyvalent metal salts include aluminum sulfate, polyaluminum chloride, iron chloride, and calcium hydroxide.

The polyvalent metal salt content in relation to the total amount of the wax, linear-chain fatty acid, linear-chain fatty acid metallic salt, hydroxy fatty acid, and amino acid compound may be 0.1% by mass or more and 10% by mass or less, preferably 0.2% by mass or more and 5% by mass or less, even more preferably 0.3% by mass or more and 1% by mass or less.

- Amounts of Constituents in the First and Second Coating Layers

The total amount of the polyamine compound, polyvinyl alcohol, polyvinylpyrrolidone, arginine compound, linear-chain fatty acid, hydroxy fatty acid, and amino acid compound in relation to the entire first coating layer may be 90% by mass or more and 100% by mass or less, preferably 95% by mass or more and 100% by mass or less.

The total amount of the wax, linear-chain fatty acid, linear-chain fatty acid metallic salt, hydroxy fatty acid, amino acid compound, and polyvalent metal salt in relation to the entire second coating layer may be 90% by mass or more and 100% by mass or less, preferably 95% by mass or more and 100% by mass or less.

External Additive(s)

The cellulosic particles according to this exemplary embodiment may have at least one external additive selected from the group consisting of silicon-containing compound particles, metallic soap particles, fatty acid ester particles, and metal oxide particles.

In particular, the cellulosic particles according to this exemplary embodiment may have at least one external additive selected from the group consisting of silicon-containing compound particles and metallic soap particles.

The presence of such external additive(s) makes more certain that the cellulosic particles according to this exemplary embodiment are highly biodegradable and exhibit little change in texture over time. Possible reasons are as follows.

Silicon-containing compound particles and metallic soap particles are able to adhere to particles larger than themselves by electrostatic adhesion and have a lower surface energy than likewise adhesive metal oxide particles and fatty acid ester particles; silicon-containing compound particles and metallic soap particles, therefore, are highly effective in improving texture. Even if some of the silicon-containing compound particles and/or metallic soap particles detach from the cellulosic particles, therefore, the associated texture loss is minor, and this leads to smaller changes in texture over time. These particles provide plenty of spaces for microorganisms to penetrate through by virtue of their particular shape, so that the superior biodegradability of the cellulose will be preserved.

For these reasons, presumably, it is more certain that the cellulosic particles are highly biodegradable and exhibit little change in texture over time.

"Silicon-containing compound particles" refers to particles containing silicon.

The silicon-containing compound particles may be particles of silicon or may be particles containing silicon and other element(s).

The silicon-containing compound particles may be silica particles.

The silica particles can be any silica-based, or $SiO_2$-based, particles, whether crystalline or amorphous. The silica particles, furthermore, may be particles produced from a raw-material silicon compound, such as waterglass or an alkoxysilane, or may be particles obtained by crushing quartz.

Using silica particles as silicon-containing compound particles makes more certain that the cellulosic particles are highly biodegradable and exhibit little change in texture over time. A possible reason is as follows.

Silica adheres to the cellulosic particles by electrostatic adhesion particularly firmly and has a particularly low surface energy; the use of silica, therefore, leads to dramatically reduced changes in texture over time and superior biodegradability for the reasons described above.

For this reason, presumably, it is more certain that the cellulosic particles are highly biodegradable and exhibit little change in texture over time.

Metallic soap particles are metallic soap-based particles.

In this context, "metallic soap-based particles" refers to particles containing 90% by mass or more metallic soap in relation to the particles themselves.

A metallic soap is a fatty acid metallic salt (metallic salt of a fatty acid), formed by a fatty acid and a metal bound together.

An example of a fatty acid metallic salt is a metallic salt of a C10 to C25 (preferably C12 to C22) fatty acid. Examples of metallic salts of C10 to C25 fatty acids include metallic salts of stearic acid, palmitic acid, lauric acid, oleic acid, linoleic acid, and ricinoleic acid.

An example of a metal in a fatty acid metallic salt is a divalent metal.

Examples of metals in fatty acid metallic salts include magnesium, calcium, aluminum, barium, and zinc.

Fatty acid ester particles are particles including fatty acid ester particles as a base component.

In this context, "particles including fatty acid ester particles as a base component" refers to particles including 90% by mass or more fatty acid ester particles in relation to the particles themselves.

An example of a fatty acid ester is the product of esterification between a C10 to C25 saturated fatty acid and a C10 to C25 alcohol.

Examples of fatty acid esters include stearyl stearate, stearyl laurate, and stearyl palmitate.

Metal oxide particles are metal oxide-based particles.

In this context, "metal oxide-based particles" refers to particles containing 90% by mass or more metal oxide in relation to the particles themselves.

The metal oxide can be an oxide of a metal other than silicon.

Examples of metal oxides include zinc oxide, magnesium oxide, iron oxide, and aluminum oxide.

For texture (specifically, feel when touched) reasons, the volume-average particle diameter of the external additive may be 1 nm or more and 100 nm or less, preferably 5 nm or more and 30 nm or less.

The volume-average particle diameter of the external additive is measured in the same way as that of the cellulose.

The amount of the external additive may be 0.1% by mass or more and 2% by mass or less of the mass of the cellulosic particles (without the external additive) as a whole.

Volume-Average Particle Diameter and Upper Geometric Standard Deviation by Number GSDv

The volume-average diameter of the cellulosic particles according to this exemplary embodiment may be 3 $\mu$m or more and less than 10 $\mu$m, preferably 4 $\mu$m or more and 9 $\mu$m or less, more preferably 5 $\mu$m or more and 8 $\mu$m or less.

Making the volume-average diameter of the cellulosic particles according to this exemplary embodiment 3 $\mu$m or more and less than 10 $\mu$m makes more certain that the cellulosic particles are highly biodegradable and exhibit little change in texture over time. Possible reasons are as follows.

If the volume-average particle diameter is 3 $\mu$m or more, the surface area of the particles is not too large; in that case the particles have good texture and are less prone to the impact of surface chipping, and, therefore, the changes in texture over time will be smaller. If the volume-average particle diameter is less than 10 $\mu$m, furthermore, the biodegradation process, which starts at the surface, tends to proceed uniformly by virtue of a moderately large surface area; the cellulosic particles, therefore, tend to be superior in biodegradability.

For these reasons, presumably, it is more certain that the cellulosic particles are highly biodegradable and exhibit little change in texture over time.

The upper geometric standard deviation by number GSDv of the cellulosic particles according to this exemplary embodiment may be 1.0 or greater and 1.7 or less, preferably 1.0 or greater and 1.5 or less, more preferably 1.0 or greater and 1.3 or less.

Making the upper geometric standard deviation by number GSDv of the cellulosic particles according to this exemplary embodiment 1.0 or greater and 1.7 or less makes more certain that the cellulosic particles are highly biodegradable and exhibit little change in texture over time. Possible reasons are as follows.

If the GSDv is 1.0 or greater and 1.7 or less, it is unlikely that residual fine particles (small particles, smaller than 3 μm) affect texture because such fine particles are scarce; the changes in texture over time, therefore, will be smaller. In that case, furthermore, it is unlikely that coarse particles (large particles, larger than 10 μm) will inhibit the biodegradation process (because the cellulosic particles break down at their surface first), and this tends to help achieve superior biodegradability.

For these reasons, presumably, it is more certain that the cellulosic particles are highly biodegradable and exhibit little change in texture over time.

The volume-average diameter and the upper geometric standard deviation GSDp of the cellulosic particles are measured as follows.

Particle diameters are measured using the LS particle size distribution analyzer "Beckman Coulter LS13 320 (Beckman Coulter)," and the cumulative distribution of particle diameters is plotted as a function of volume starting from the smallest diameter; then the particle diameter at which the cumulative percentage is 50% is determined as the volume-average particle diameter.

Separately, the cumulative distribution of particle diameters is plotted as a function of volume starting from the smallest diameter, and the particle diameters at which the cumulative percentage is 50% and 84% are defined as the number-average particle diameter, D50v, and particle diameter D84v by number, respectively. The upper geometric standard deviation by number GSDv is calculated according to the equation $GSDv = (D84v/D50v)^{1/2}$.

Sphericity

The sphericity of the cellulosic particles according to this exemplary embodiment may be 0.90 or greater, preferably 0.95 or greater, more preferably 0.97 or greater.

Making the sphericity of the cellulosic particles according to this exemplary embodiment 0.90 or greater makes more certain that the cellulosic particles are highly biodegradable and exhibit little change in texture over time. Possible reasons are as follows.

If the sphericity is 0.9 or greater, the changes in texture over time will be smaller because the impact of surface defects, if any, will be minimized. In that case, furthermore, the particles tend to be superior in biodegradability, too, because the distance from the surface to the inner core of the particles, for which microorganisms need to go to decompose the particles, is the shortest.

For these reasons, presumably, it is more certain that the cellulosic particles are highly biodegradable and exhibit little change in texture over time.

The sphericity is given by (circumference of the equivalent circle)/(circumference) [(circumference of a circle having the same projected area as the particle's image)/(circumference of the particle's projected image)]. Specifically, the sphericity is a value measured by the following method.

First, a portion of the cellulosic particles of interest is sampled by aspiration in such a manner that it will form a flat stream, and this flat stream is photographed with a flash to capture the figures of the particles in a still image; then the sphericity is determined by analyzing the particle images using a flow particle-image analyzer (Sysmex Corp. FPIA-3000). The number of particles sampled in the determination of the sphericity is 3500.

If the cellulosic particles have an external additive, the cellulosic particles of interest are dispersed in water containing a surfactant first; then the external additive is removed through sonication, and the sonicated particles are subjected to the measurement.

Surface Smoothness

The surface smoothness of the cellulosic particles according to this exemplary embodiment may be 80% or higher, preferably 82% or higher and 99% or lower, more preferably 84% or higher and 98% or lower.

Making the surface smoothness of the cellulosic particles according to this exemplary embodiment 80% or higher helps ensure that the cellulosic particles are highly biodegradable and exhibit little change in texture over time. Possible reasons are as follows.

If the surface smoothness is 80% or higher, the changes in texture over time will be smaller because any instances of chipping of the surface of the particles scarcely have impact on texture by virtue of the overall smoothness of the particles. In that case, furthermore, the cellulosic particles tend to be superior in biodegradability because large-sized microorganisms (some kinds of biodegrading microorganisms are relatively large in size) can get access to the surface of the particles.

For these reasons, presumably, it is likely that the cellulosic particles are highly biodegradable and exhibit little change in texture over time.

The surface smoothness is measured through a procedure as described below.

An SEM image (magnification, 5,000 times) of the cellulosic particles, taken with a scanning electron microscope (SEM), is observed, and the smoothness M of the individual cellulosic particles is calculated according to the equation below. The arithmetic mean smoothness M of any ten or more cellulosic particles is reported as the surface smoothness. The closer the smoothness M is to 1, the closer the surface of the cellulosic particles is to smoothness.

$$M = (1-(S3)/(S2)) \times 100$$

In this equation, S2 denotes the area of the cellulosic particle in the image (projected area), and S3 denotes, when the cellulosic particle in the image is superimposed on a circle having a projected area equal to S2, the sum of "the area outside the outline of the circle having a projected area equal to S2 and inside the outline of the cellulosic particle in the image" and "the area inside the outline of the circle having a projected area equal to S2 and outside the outline of the cellulosic particle in the image."

The superposition of the cellulosic particle in the image on a circle having a projected area equal to S2 is done as follows.

The cellulosic particle in the image is superimposed on the circle having a projected area equal to S2 in such a manner as to maximize the area of overlap between the two images (the area inside the outline of the circle having a projected area equal to S2 and inside the outline of the cellulosic particle in the image).

Method for Producing the Cellulosic Particles

A method for producing the cellulosic particles includes a step of producing a particle precursor containing a cellulose acylate (particle precursor production step) and a step of saponifying the cellulose acylate contained in the particle precursor (saponification step).

Particle Precursor Production Step

A particle precursor containing a cellulose acylate is produced by any of methods (1) to (5) below.

(1) Kneading and milling, in which the ingredients are kneaded together, and the resulting mixture is milled and classified to give a granular material

(2) A dry process, in which the shape of particles of the granular material obtained by kneading and milling is changed with the help of a mechanical impact force or thermal energy

(3) Aggregation and coalescence, in which dispersions of particles of the ingredients are mixed together, and the particles in the mixed dispersion are caused to aggregate and fused together under heat to give a granular material

(4) Dissolution and suspension, in which a solution of the ingredients in an organic solvent is suspended in an aqueous medium to form a granular material containing the ingredients

(5) Kneading and dissolution, in which the ingredients and a binder are kneaded together, the resulting mixture is pelletized by extrusion, and the resulting pellets are stirred in a solvent for the binder to form a granular material

In this context, a cellulose acylate is a cellulose derivative in which at least one of the hydroxy groups of cellulose has been replaced with an aliphatic acyl group (acylated). Specifically, a cellulose acylate is a cellulose derivative in which at least one of the hydroxy groups of cellulose has been replaced with -CO-R$^{AC}$ (R$^{AC}$ represents an aliphatic hydrocarbon group.).

Saponification Step

Then the cellulose acylate contained in the particle precursor is saponified.

Through this step, the aliphatic acyl group(s) of the cellulose acylate is hydrolyzed, and the cellulose turns into cellulose.

The saponification step is performed by, for example, adding sodium hydroxide to a dispersion of the particle precursor and stirring the dispersion.

Coating Layer Formation Step

If coated cellulosic particles are produced, the production method may include a step of forming the coating layer (coating layer formation step) after the above saponification step.

If the coating layer formation step is performed, the coating layer is formed using the particles obtained through the above saponification step as core particles.

First, an aqueous dispersion in which the core particles are dispersed is prepared. The core particles may be cleaned with acid before the preparation of the aqueous dispersion.

Then the aqueous dispersion in which the core particles are dispersed is mixed with an aqueous solution containing the compound(s) that will form the first coating layer. This causes, for example, hydroxyl groups of the resin contained in the core particles to react with, for example, amine sites, carboxyl groups, or amino groups of the surface-treating polymer(s) or to form hydrogen bonds with hydroxyl groups of the polymer(s), and this produces the first coating layer. Then the aqueous dispersion in which the core particles with the first coating layer formed thereon are dispersed is mixed with an emulsion containing the compound(s) that will form the second coating layer. Through this, the second coating layer is formed.

Then the cellulosic particles having coating layers are removed from the mixture. The removal of the cellulosic particles having coating layers is done by, for example, filtering the mixture. The removed cellulosic particles having coating layers may be washed with water. This helps eliminate unreacted residue of the surface-treating polymer(s). Then the cellulosic particles having coating layers are dried, giving cellulosic particles according to this exemplary embodiment.

Addition Step

External additive(s) may be added to the resulting cellulosic particles.

An example of an addition step is a treatment in which the external additive(s) is added to the cellulosic particles using equipment like a mixing mill, V-blender, Henschel mixer, or Lödige mixer.

Applications

Applications of the cellulosic particles according to this exemplary embodiment include granular materials for use as cosmetics, a rolling agent, an abrasive, a scrubbing agent, display spacers, a material for bead molding, light-diffusing particles, a resin-strengthening agent, a refractive index control agent, a biodegradation accelerator, a fertilizer, water-absorbent particles, toner particles, and anti-blocking particles.

An application of the cellulosic particles according to this exemplary embodiment may be cosmetics.

An application of the cellulosic particles according to this exemplary embodiment may be a cosmetic additive in particular.

Superior in flexibility, the cellulosic particles according to this exemplary embodiment, if used as a cosmetic additive, help the cosmetic product to spread well on the skin to which it is applied.

The cellulosic particles according to this exemplary embodiment can be applied as cosmetic additives, for example to base makeup cosmetics (e.g., foundation primer, concealer, foundation, and face powder); makeup cosmetics (e.g., lipstick, lip gloss, lip liner, blush, eyeshadow, eyeliner, mascara, eyebrow powder, nail products, and nail care cosmetics); and skincare cosmetics (e.g., face wash, facial cleanser, toner, milky lotion, serum, face packs, face masks, and cosmetics for the care of the eye and mouth areas).

The resin particles according to this exemplary embodiment may be used as a cosmetic additive to makeup cosmetics in particular, because cosmetic additives to makeup cosmetics can need to be flexible and biodegradable.

Examples

Examples will now be described, but no aspect of the present disclosure is limited to these examples. In the following description, "parts" and "%" are all by mass unless stated otherwise.

Preparation of Materials

The following materials are prepared.

Cellulose Acylates

- Cel1: Daicel "L-20"; cellulose acetate; number-average molecular weight, 47000.
- Cel2: Daicel "L-50"; cellulose acetate; number-average molecular weight, 58000.
- Cel3: Eastman Chemical "CAP482-20"; cellulose acetate propionate; number-average molecular weight, 75000.

- Cel4: Eastman Chemical "CAB381-20"; cellulose acetate butyrate; number-average molecular weight, 70000.
- Cel5: Eastman Chemical "CA398-6"; cellulose acetate; number-average molecular weight, 35000.
- Cel6: Eastman Chemical "CAP482-0.5"; cellulose acetate propionate; number-average molecular weight, 25000.
- Cel7: Eastman Chemical "CAP-504-0.2"; cellulose acetate propionate; number-average molecular weight, 15000.

Polyamine Compounds

- Fir1: Nippon Shokubai "EPOMIN SP-003"; polyethyleneimine; molecular weight, 300
- Fir2: Nippon Shokubai "EPOMIN SP-006"; polyethyleneimine; molecular weight, 600
- Fir3: Nippon Shokubai "EPOMIN SP-012"; polyethyleneimine; molecular weight, 1200
- Fir4: Nippon Shokubai "EPOMIN SP-018"; polyethyleneimine; molecular weight, 1800
- Fir5: Nippon Shokubai "EPOMIN SP-200"; polyethyleneimine; molecular weight, 10000
- Fir6: Nippon Shokubai "EPOMIN HM-2000"; polyethyleneimine; molecular weight, 30000
- Fir7: Nippon Shokubai "EPOMIN P-1000"; polyethyleneimine; molecular weight, 70000
- Fir8: Nittobo Medical "PAA-01"; polyallylamine; molecular weight, 1600
- Fir9: Nittobo Medical "PAA-03 "; polyallylamine; molecular weight, 3000
- Fir10: Nittobo Medical "PAA-05"; polyallylamine; molecular weight, 5000
- Fir11: Nittobo Medical "PAA-08"; polyallylamine; molecular weight, 8000
- Fir12: Nittobo Medical "PAA-15C"; polyallylamine; molecular weight, 15000
- Fir13: Nittobo Medical "PAA-25"; polyallylamine; molecular weight, 25000
- Fir14: Mitsubishi Chemical "Polyvinylamine," polyvinylamine
- Fir15: JNC "Polylysine 10," polylysine
- Fir16: Ichimaru Pharcos "Polylysine 10," polylysine
- Fir31: BASF Japan "Dehyquart H81," PEG-15 cocopolyamine

Polyvinyl Alcohol and Polyvinylpyrrolidone

- Fir17: Mitsubishi Chemical "GOHSENOL N-300," polyvinyl alcohol
- Fir18: Nippon Shokubai "K-30," polyvinylpyrrolidone

Linear-Chain Fatty Acids

- Fir19: NOF "NAA-222S," behenic acid (C22)
- Fir20: FUJIFILM Shonan Wako Junyaku "Arachidic Acid," arachidic acid (C20)
- Fir21: FUJIFILM Shonan Wako Junyaku "Palmitic Acid," palmitic acid (C14)
- Fir22: FUJIFILM Shonan Wako Junyaku "Lauric Acid," lauric acid (C12)
- Fir23: FUJIFILM Shonan Wako Junyaku "Lignoceric Acid," lignoceric acid (C24)

Hydroxy Fatty Acids

- Fir24: Itoh Oil Chemicals "12-Hydroxystearic Acid," hydroxystearic acid
- Fir25: NOF, "Hydrogenated Castor Oil Fatty Acid," a hydrogenated castor oil fatty acid Amino Acid Compound
- Fir26: Ajinomoto "AMIHOPE LL," lauroyl lysine

Arginine Compounds

- Fir32: Nippon Rika "L-Arginine"
- Fir33: Ajinomoto "L-Arginine (C grade)"

- Fir34: Ajinomoto "CAE," PCA ethyl cocoyl arginate

Linear-Chain Fatty Acid Metallic Salt

- Fir41: NOF "CALCIUM STEARATE VEGETABLE," calcium stearate Waxes
- Sec1: Senka "CN-100," carnauba wax
- Sec2: Toa Kasei "TOWAX-1F3," carnauba wax
- Sec3: Toa Kasei "TOWAX-1F6," carnauba wax
- Sec4: Toa Kasei "TOWAX-1F8," carnauba wax
- Sec5: Toa Kasei "TOWAX-1F12," carnauba wax
- Sec6: Toa Kasei "TOWAX-5B2," carnauba wax
- Sec7: Toa Kasei "TOWAX-1B4," carnauba wax
- Sec8: Toa Kasei "TOWAX-4F2," candelilla wax
- Sec9: Toa Kasei "TOWAX-4F3," candelilla wax
- Sec10: Toa Kasei "TOWAX-4F4," candelilla wax
- Sec11: Toa Kasei "TOWAX-6B2," *Rosa damascena* flower wax
- Sec12: Toa Kasei "TOWAX-6F2," sunflower seed wax
- Sec13: Kokura Gosei Kogyo, rice bran wax
- Sec14: Boso Oil and Fat "SS-1," rice bran wax
- Sec15: Nisshin OilliO "COSMOL 222," diisostearyl malate

Polyvalent Metal Salts

- Sec21: FUJIFILM Wako Pure Chemical, aluminum sulfate
- Sec22: FUJIFILM Wako Pure Chemical, polyaluminum chloride
- Sec23: FUJIFILM Wako Pure Chemical, iron chloride
- Sec24: FUJIFILM Wako Pure Chemical, calcium hydroxide

External Additives

Silicon-Containing Compound Particles

- Sur1: Nippon Aerosil "AEROSIL R972," silica dimethyl silylate particles, average diameter = 16 nm
- Sur2: Nippon Aerosil "AEROSIL RY200S," silica dimethicone silylate particles, average diameter = 12 nm

Metallic Soap Particles

- Sur3: NOF "MZ-2," zinc stearate particles, volume-average diameter = 1.5 $\mu$m
- Sur4: NOF "Magnesium Stearate S," magnesium stearate particles, volume-average diameter = 1 $\mu$m

Fatty Acid Ester Particles

- Sur6: Kao "EXCEPARL SS," stearyl stearate particles, volume-average diameter = 1 $\mu$m

Metal Oxide Particles

- Sur7: Sakai Chemical "FINEX-50," zinc oxide particles, volume-average diameter = 1.5 µm

The volume-average particle diameters of the external additives are measured through the same procedure as the volume-average diameters of the cellulosic particles.

Example 1

Particle Precursor Production Step

One hundred thirty parts of cellulose acylate Cel1 is dissolved completely in 870 parts of ethyl acetate. The resulting solution is added to a water-based liquid containing 50 parts of calcium carbonate and 500 parts of purified water, and the resulting mixture is stirred for 3 hours (hereinafter referred to as "the first stirring time"). A dispersion of 4 parts of carboxymethyl cellulose (hereinafter also referred to as "CMC") and 200 parts methyl ethyl ketone in 600 parts of purified water is added, and the resulting mixture is stirred for 5 minutes using a high-speed emulsifier. Ten parts of sodium hydroxide is added, and the resulting mixture is heated to 80°C and stirred for 3 hours so that the ethyl acetate and the methyl ethyl ketone will be removed. The same amount of diluted hydrochloric acid as the sodium hydroxide is added, the residue is collected by filtration, and the collected solids are dispersed once again in purified water; this gives a particle precursor dispersion (solids concentration, 10%).

Saponification Step

A mixture obtained by adding 17.5 parts of a 20% aqueous solution of sodium hydroxide to 500 parts of the particle precursor dispersion is stirred for 6 hours at a saponification temperature of 30°C. After the pH is adjusted to 7 with hydrochloric acid, the saponified slurry is cleaned by repeated filtration and washing until the electrical conductivity of the filtrate is 10 µs/cm or less; this gives cellulosic particles.

Examples 2 to 7

Cellulosic particles are obtained through the same procedure as in Example 1, except that in the particle precursor production step, the cellulose acylate species is as in Table 1.

Example 8

Particle Precursor Production and Saponification Steps

Cellulosic particles are obtained through the same procedure as in Example 1. Coating Layer Formation Step

One thousand parts of the cellulosic particles, which are core particles, and 10000 parts of deionized water are mixed together; this gives a core particle dispersion. Five parts of Fir16, which will form the first coating layer, is added to the core particle dispersion, and the resulting mixture is stirred for 1 hour so that the compound will form a coating layer. The coated cellulosic particles are cleaned by repeated filtration and washing until the electrical conductivity of the filtrate is 10 µs/cm or less; this gives coated cellulosic particles.

Examples 9 to 25

Coated cellulosic particles are obtained through the same procedure as in Example 8, except that in the coating layer formation step, the species of the compound that will form the first coating layer ("First-layer compound" in Table 1) is as in Table 1.

Example 26

Particle Precursor Production and Saponification Steps

Cellulosic particles are obtained through the same procedure as in Example 1. Coating Layer Formation Step

One thousand parts of the cellulosic particles, which are core particles, and 10000 parts of deionized water are mixed together; this gives a core particle dispersion. Five parts of Fir16, which will form the first coating layer, is added to the core particle dispersion, and the resulting mixture is stirred for 1 hour so that the compound will form a first coating layer;

this gives a dispersion of cellulosic particles having a first coating layer.

Then an emulsion for the formation of the second coating layer is prepared by mixing 6 parts of wax Sec1 and 50 parts of purified water together using a high-speed emulsifier.

All of the emulsion for the formation of the second coating layer is added to the dispersion of cellulosic particles having a first coating layer, and the resulting mixture is stirred for 24 hours so that the wax will form the second coating layer; this gives a dispersion of cellulosic particles having first and second coating layers.

The cellulosic particles having first and second coating layers are cleaned by repeated filtration and washing until the electrical conductivity of the filtrate is 10 μs/cm or less; this gives cellulosic particles having first and second coating layers.

Examples 28 to 41

Cellulosic particles having first and second coating layers are obtained through the same procedure as in Example 26, except that in the coating layer formation step, the wax species is as in Table 1.

Examples 42 to 44

Cellulosic particles having first and second coating layers are obtained through the same procedure as in Example 26, except that in the coating layer formation step, the amount of the compound that will form the first coating layer and the amount of wax are as in Table 1.

Examples 45

Particle Precursor Production, Saponification, and Coating Layer Formation Steps

Cellulosic particles having first and second coating layers are obtained through the same procedure as in Example 26.

Addition Step

A 0.6-part portion of external additive Sur1 is added to 30 parts of the cellulosic particles having first and second coating layers, and the ingredients are mixed together in a mixing mill (WONDER CRUSHER, Osaka Chemical); this gives cellulosic particles having an external additive.

Examples 46 to 48 and 50 to 53

Cellulosic particles having an external additive are obtained through the same procedure as in Example 44, except that in the addition step, the external additive and its amount are as in Table 1.

Examples 54 to 61

Cellulosic particles having an external additive are obtained through the same procedure as in Example 26, except that in the particle precursor production step, the amount of calcium carbonate, the first stirring time, the amount of carboxymethyl cellulose, and the amount of sodium hydroxide are as in Table 1.

Examples 64 and 65

Coated cellulosic particles are obtained through the same procedure as in Example 26 or 45, except that the coating layer formation step is done without the process of adding 5 parts of Fir16, the compound for the formation of the first coating layer, to the core particle dispersion and stirring the resulting mixture for 1 hour.

Examples 66 to 69

Cellulosic particles having an external additive are obtained through the same procedure as in Example 45, except that in the coating layer formation step, the wax species is as in Table 1 and that in preparing the emulsion for the formation of the second coating layer, the polyvalent metal salt specified in Table 1, its amount being as in Table 1, is added together with the wax and the purified water.

Examples 70 to 84

Cellulosic particles are obtained through the same procedure as in the above Examples, except that the parameters are changed to those indicated in Table 1.

Comparative Examples 1 to 4

The following particles are used as cellulosic particles of the comparative examples.

Comparative Example 1: CELLULOBEADS D10 (Daito Kasei, cellulosic particles containing cellulose as their base constituent. No coating layer and no external additive.)
Comparative Example 2: OTS-0.5A CELLULOBEADS D10 (Daito Kasei, cellulosic particles having a cellulose-based core particle and a coating layer containing triethoxyoctylsilane. No external additive.)
Comparative Example 3: S-STM CELLULOBEADS D-5 (Daito Kasei, cellulosic particles having a cellulose-based core particle and a coating layer containing magnesium stearate. No external additive.)
Comparative Example 4: CELLUFLOW C25 (JNC, cellulosic particles containing cellulose as their base constituent. No coating layer and no external additive.)

Comparative Example 5

Cellulosic particles are obtained according to the procedure described in Example 1 in Japanese Patent No. 6872068. These cellulosic particles contain cellulose as their base constituent and have no external additive. The specific production process is as follows.

An oil phase is prepared by dissolving 250 parts by mass of diacetyl cellulose (CA398-3, Eastman Chemical) in 2500 parts by mass of ethyl acetate. A water phase is prepared by dissolving 200 parts by mass of polyvinyl alcohol in 2300 parts by mass of deionized water. The prepared water phase is mixed with the oil phase, and the resulting mixture is stirred at 1000 rpm for 3 minutes using a dissolver. The mixture is further stirred at 1800 rpm for 10 minutes using a dissolver to give a suspension in which the oil phase is dispersed uniformly.

While the resulting suspension is stirred at 500 rpm, 112500 parts by mass of deionized water is introduced over 75 minutes; this gives a dispersion of resin particles. The resin particles are collected by filtration, washed, and then stirred in deionized water. After filtration and washing, the resulting resin particles are dispersed in 2500 parts by mass of deionized water. Sodium hydroxide is added to make the pH 13.0 or below, the dispersion is heated to 60°C for hydrolysis at the same time, and the dispersion is neutralized with hydrochloric acid. The product is collected by filtration, washed, and then immersed in deionized water. After filtration and washing, the solids are dried and crushed; this gives cellulosic particles.

Comparative Example 6

Cellulosic particles are obtained according to the procedure described in Example 2 in Japanese Patent No. 6872068. These cellulosic particles contain cellulose as their base constituent and have no external additive. The specific production process is as follows.

An oil phase is prepared by dissolving 250 parts by mass of cellulose acetate propionate (CAP504-0.2, Eastman Chemical) in 1000 parts by mass of ethyl acetate. A water phase is prepared by dissolving 100 parts of polyvinyl alcohol in 1088 parts of deionized water and stirring the resulting solution with 62.5 parts of ethyl acetate. The prepared water phase is mixed with the oil phase, and the resulting mixture is stirred at 1000 rpm for 3 minutes using a dissolver. The mixture is further stirred at 1500 rpm for 5 minutes to give a suspension in which oil droplets are dispersed uniformly.

While the suspension is stirred at 500 rpm, 21250 parts by mass of deionized water is introduced over 60 minutes; this gives a dispersion of resin particles. The resin particles are collected by filtration, washed, immersed in deionized water, and stirred. After filtration and washing, the solids are dried and crushed into resin particles. The resulting resin particles are dispersed in 5000 parts by mass of deionized water. Sodium hydroxide is added to make the pH 13.0 or below, the dispersion is heated to 40°C for hydrolysis, and then the dispersion is neutralized with acetic acid. The product is collected by filtration and washed; this gives cellulosic particles.

Comparative Example 7

Cellulosic particles are obtained according to the procedure described in Example 1 in Japanese Unexamined Patent Application Publication No. 2021-021044. These cellulosic particles contain cellulose as their base constituent and have no coating layer and no external additive. The specific production process is as follows.

A 4.8-g portion of cyclohexanone is stirred with 0.2 g of diacetyl cellulose (L20, Daicel). The resulting mixture is further stirred at 60°C for 3 hours to give a 4% by mass solution of diacetyl cellulose; this solution is the dispersed phase.

Fifty grams of purified water is stirred with 0.1 g of sodium dodecylbenzenesulfonate and 3.5 g of cyclohexanone. The resulting mixture is warmed to 60°C to give an aqueous medium; this aqueous medium is the continuous phase. The dispersed phase, preheated to 60°C, and the continuous phase, also preheated to 60°C, are put into different inlets of a rotational cylinder emulsifier (cylinder outer diameter, 78 mm; cylinder length, 215 mm; cylinder inner diameter, 80 mm; clearance, 1 mm; Tipton) at 1 mL/min using a syringe pump (high-pressure microfeeder JP-H, Furue Science) and at 10 mL/min using a plunger pump (NP-KX-840, Nihon Seimitsu Kagaku), respectively, and emulsified at a cylinder rotational frequency of 2000 rpm for an emulsification period of 138 seconds to give an oil-in-water emulsion.

This oil-in-water emulsion is cooled to 5°C and fed to a double-tube merger, and the diacetyl cellulose is precipitated by feeding purified water at 10 mL/min; this gives a solution of particle slurry.

The resulting diacetyl cellulose particles are put into a mixture of 7 parts by mass of a 55% by mass aqueous solution of methanol and 3.5 parts by mass of a 20% by mass aqueous solution of sodium hydroxide (concentrations based on the diacetyl cellulose particles), and the resulting mixture is stirred at 35°C for 20 hours so that the diacetyl cellulose particles will be saponified; this gives cellulosic particles.

Comparative Example 8

Cellulosic particles are obtained according to the procedure described in Example 1 in Japanese Unexamined Patent Application Publication No. 2021-021045. These cellulosic particles contain cellulose as their base constituent and have no coating layer and no external additive. The specific production process is as follows.

Diacetyl cellulose (L20, Daicel) is added to 64 g of ethyl acetate and 16 g of acetone, and the resulting mixture is stirred at 50°C for 3 hours or longer to give a 10% by mass diacetyl cellulose solution.

This solution is poured into 82.8 g of purified water at 50°C containing 0.18 g of sodium dodecylbenzenesulfonate and 6.2 g of ethyl acetate, and the resulting mixture is stirred at a rotational frequency of 300 rpm for 10 minutes; this gives a crude emulsion. A porous membrane (a cylindrical SPG membrane having an outer diameter of 10 mm, a thickness of 1 mm, and a pore diameter of 50 $\mu$m; SPG Technology) is immersed in a container holding 331.2 g of purified water at 50°C containing 0.71 g of sodium dodecylbenzenesulfonate and 24.9 g of ethyl acetate, and the container in which the crude emulsion has been prepared is coupled to the inside of this porous membrane. The crude emulsion is forced through the membrane by applying a pressure of 100 kPa to the container in which the crude emulsion has been prepared; membrane emulsification induced by this gives an oil droplet-in-water emulsion.

This emulsion is cooled, and when its temperature is 20°C, 444 mL of purified water is added dropwise; this gives spherical diacetyl cellulose particles. Then the dispersion is centrifuged and filtered, and the residual diacetyl cellulose particles are washed thoroughly with plenty of water and collected by filtration; this yields 2.8 g of diacetyl cellulose particles.

The resulting diacetyl cellulose particles are put into a mixture of a 55% aqueous solution of methanol (7 parts by mass) and a 20% by mass aqueous solution of sodium hydroxide (3.5 parts by mass) (concentrations based on the diacetyl cellulose particles), and the resulting mixture is stirred at 35°C for 20 hours so that the diacetyl cellulose will be saponified; this gives cellulosic particles.

Comparative Example 9

CELLUFLOW TA25 (JNC, diacetyl cellulose particles. No coating layer and no external additive.) is used as cellulosic particles of Comparative Example 9.

Comparative Example 10

Cellulosic particles are obtained according to the procedure described in Example 1 in Japanese Patent No. 6921293. The specific production process is as follows.

An oil phase is prepared by dissolving 150 parts of diacetyl cellulose (trade name "CA-398-6," Eastman Chemical; acetyl content, 39.8%) in 1,350 parts of ethyl acetate (solubility in water, 8 g/100 g). A water phase is prepared by dissolving 100 parts of polyvinyl alcohol in 1,250 parts of deionized water. The prepared water phase is mixed with the oil phase, and the resulting mixture is stirred at 1,000 rpm for 3 minutes using a dissolver. The mixture is further stirred at 2,000 rpm for 10 minutes using a dissolver, giving a suspension in which oil droplets are dispersed uniformly. The volume-average diameter of the oil droplets measured by optical microscope observation and image analysis is 18 $\mu$m.

While the resulting suspension is stirred at 500 rpm using a dissolver, 42,000 parts of deionized water is introduced over 90 minutes; this gives a dispersion of resin particles. After filtration and washing, the resin particles are deflocculated in deionized water and stirred. The resin particles are collected by filtration, washed, and dispersed in 2,500 parts of deionized water. Sodium hydroxide is added to make the pH 13.0 or below, and the dispersion is heated to 50°C for hydrolysis at the same time. After the end of the hydrolysis, the dispersion is neutralized with hydrochloric acid. The product is collected by filtration, washed, and then deflocculated in deionized water. After filtration and washing, the solids are dried and crushed; this gives core beads having a median diameter (D50) of 9 μm.

Fifty grams of the resulting core beads and 1.5 g of zinc stearate (trade name "SPZ-100F," Sakai Chemical Industry; a powder of sheet-shaped particles; average particle diameter, 0.4 μm; thickness, 0.1 μm; aspect ratio, 3) are put into a small-sized mixer. The materials are dry-mixed for 3 minutes so that the surface of the core beads will be treated with the zinc stearate; this gives resin beads.

The resulting resin beads are used as cellulosic particles of Comparative Example 10. Comparative Example 11 Cellulosic particles are obtained according to the procedure described in Example 2 in Japanese Patent No. 6921293. The specific production process is as follows.

Resin beads are obtained in the same way as in Example 1 in Japanese Patent No. 6921293, except that the zinc stearate is replaced with 2.5 g of magnesium stearate (trade name "SPX-100F," Sakai Chemical Industry; a powder of sheet-shaped particles; average particle diameter, 0.7 μm; thickness, 0.1 μm; aspect ratio, 4).

The resulting resin beads are used as cellulosic particles of Comparative Example 11. Examples 101 to 124

Coated cellulosic particles are obtained through the same procedure as in the above Examples, except that the parameters are changed to those indicated in Table 1.

Evaluations

The following characteristics of the cellulosic particles obtained in the Examples and Comparative Examples are measured according to the methods described previously herein.

- Five-day percentage biodegradation measured as per JIS K6950:2000 ("Biodegradation, 5 days" in the tables)
- Sixty-day percentage biodegradation measured as per JIS K6950:2000 ("Biodegradation, 60 days" in the tables)
- Volume-average diameter of the cellulosic particles ("Particle diameter" in the tables)
- Upper geometric standard deviation by number of the cellulosic particles ("GSDv" in the tables)
- Sphericity of the cellulosic particles
- Number-average molecular weight of the cellulose in the cellulosic particles ("Mn" in the tables)
- Surface smoothness of the cellulosic particles

Texture Evaluations

Smoothness

For deterioration in smoothness over time, ten female testers spread the particles on the back of their hand and grade their feeling from 1 for "unsmooth" to 10 for "smooth"; the average rate of the ten testers is the score. This test is performed after the freshly produced particles are left at room temperature for 24 hours and in a temperature-controlled chamber at a temperature of 50°C and a relative humidity of 85% rh for 96 hours (initial and follow-up tests, respectively), and the difference between the grades in the initial and follow-up tests is the deterioration in smoothness over time.

Moist Sensation

For deterioration in moist sensation over time, ten female testers spread the particles on the back of their hand and grade their feeling from 1 for "too dry" to 10 for "moist"; the average rate of the ten testers is the score. This test is performed after the freshly produced particles are left at room temperature for 24 hours and in a temperature-controlled chamber at a temperature of 50°C and a relative humidity of 85% rh for 96 hours (initial and follow-up tests, respectively), and the difference between the grades in the initial and follow-up tests is the deterioration in moist sensation over time.

Softness

For deterioration in softness over time, ten female testers spread the particles on the back of their hand and grade their feeling from 1 for "hard and difficult to spread" to 10 for "very soft"; the average rate of the ten testers is the score. This test is performed after the freshly produced particles are left at room temperature for 24 hours and in a temperature-controlled chamber at a temperature of 50°C and a relative humidity of 85% rh for 96 hours (initial and follow-up tests, respectively), and the difference between the grades in the initial and follow-up tests is the deterioration in smoothness over time.

[Table 1-1]

| Table 1-1 Particle Production Parameters | | | | | | | | | |
|---|---|---|---|---|---|---|---|---|---|
| | | Particle precursor production step | | | | | Saponification step | | |
| | Particle number | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 1 | Par01 | Cell | 50 | 3 | 4 | 10 | 17.5 | 30 | 6 |
| Example 2 | Par02 | Cel2 | 50 | 3 | 4 | 10 | 17.5 | 30 | 6 |
| Example 3 | Par03 | Cel3 | 50 | 3 | 4 | 10 | 17.5 | 30 | 6 |
| Example 4 | Par04 | Cel4 | 50 | 3 | 4 | 10 | 17.5 | 30 | 6 |
| Example 5 | Par05 | Cel5 | 50 | 3 | 4 | 10 | 17.5 | 30 | 6 |
| Example 6 | Par06 | Cel6 | 50 | 3 | 4 | 10 | 17.5 | 30 | 6 |
| Example 7 | Par07 | Cel7 | 50 | 3 | 4 | 10 | 17.5 | 30 | 6 |
| Example 8 | Par08 | Cell | 50 | 3 | 4 | 10 | 17.5 | 30 | 6 |
| Example 9 | Par09 | Cell | 50 | 3 | 4 | 10 | 17.5 | 30 | 6 |
| Example 10 | Par010 | Cell | 50 | 3 | 4 | 10 | 17.5 | 30 | 6 |
| Example 11 | Par011 | Cell | 50 | 3 | 4 | 10 | 17.5 | 30 | 6 |
| Example 12 | Par012 | Cell | 50 | 3 | 4 | 10 | 17.5 | 30 | 6 |
| Example 13 | Par013 | Cell | 50 | 3 | 4 | 10 | 17.5 | 30 | 6 |
| Example 14 | Par014 | Cell | 50 | 3 | 4 | 10 | 17.5 | 30 | 6 |
| Example 15 | Par015 | Cell | 50 | 3 | 4 | 10 | 17.5 | 30 | 6 |
| Example 16 | Par016 | Cell | 50 | 3 | 4 | 10 | 17.5 | 30 | 6 |
| Example 17 | Par017 | Cell | 50 | 3 | 4 | 10 | 17.5 | 30 | 6 |
| Example 18 | Par018 | Cell | 50 | 3 | 4 | 10 | 17.5 | 30 | 6 |
| Example 19 | Par019 | Cell | 50 | 3 | 4 | 10 | 17.5 | 30 | 6 |
| Example 20 | Par020 | Cell | 50 | 3 | 4 | 10 | 17.5 | 30 | 6 |
| Example 21 | Par021 | Cell | 50 | 3 | 4 | 10 | 17.5 | 30 | 6 |
| Example 22 | Par022 | Cell | 50 | 3 | 4 | 10 | 17.5 | 30 | 6 |
| Example 23 | Par023 | Cell | 50 | 3 | 4 | 10 | 17.5 | 30 | 6 |

| | | Particle precursor production step | | | | | Saponification step | | |
|---|---|---|---|---|---|---|---|---|---|
| | Particle number | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 24 | Par024 | Cell | 50 | 3 | 4 | 10 | 17.5 | 30 | 6 |
| Example 25 | Par025 | Cell | 50 | 3 | 4 | 10 | 17.5 | 30 | 6 |
| Example 26 | Par026 | Cell | 50 | 3 | 4 | 10 | 17.5 | 30 | 6 |
| Example 28 | Par028 | Cell | 50 | 3 | 4 | 10 | 17.5 | 30 | 6 |
| Example 29 | Par029 | Cell | 50 | 3 | 4 | 10 | 17.5 | 30 | 6 |
| Example 30 | Par030 | Cell | 50 | 3 | 4 | 10 | 17.5 | 30 | 6 |
| Example 31 | Par031 | Cell | 50 | 3 | 4 | 10 | 17.5 | 30 | 6 |
| Example 32 | Par032 | Cell | 50 | 3 | 4 | 10 | 17.5 | 30 | 6 |
| Example 33 | Par033 | Cell | 50 | 3 | 4 | 10 | 17.5 | 30 | 6 |
| Example 34 | Par034 | Cell | 50 | 3 | 4 | 10 | 17.5 | 30 | 6 |
| Example 35 | Par035 | Cell | 50 | 3 | 4 | 10 | 17.5 | 30 | 6 |
| Example 36 | Par036 | Cell | 50 | 3 | 4 | 10 | 17.5 | 30 | 6 |
| Example 37 | Par037 | Cell | 50 | 3 | 4 | 10 | 17.5 | 30 | 6 |
| Example 38 | Par038 | Cell | 50 | 3 | 4 | 10 | 17.5 | 30 | 6 |
| Example 39 | Par039 | Cell | 50 | 3 | 4 | 10 | 17.5 | 30 | 6 |
| Example 40 | Par040 | Cell | 50 | 3 | 4 | 10 | 17.5 | 30 | 6 |
| Example 41 | Par041 | Cell | 50 | 3 | 4 | 10 | 17.5 | 30 | 6 |
| Example 42 | Par042 | Cell | 50 | 3 | 4 | 10 | 17.5 | 30 | 6 |
| Example 43 | Par043 | Cell | 50 | 3 | 4 | 10 | 17.5 | 30 | 6 |

Table 1-1 Particle Production Parameters

EP 4 223 273 B1

[Table 1-2]

| | Particle number | Coating layer formation step | | | | | | Addition step | |
|---|---|---|---|---|---|---|---|---|---|
| Table 1-2 Particle Production Parameters | | First-layer compound | | Second-layer compound, wax | | Second-layer compound, polyvalent metal salt | | External additive | |
| | | Species | Amount (parts) | Species | Amount (parts) | Species | Amount (parts) | Species | Amount (parts) |
| Example 1 | Par0 1 | | | | | | | | |
| Example 2 | Par02 | | | | | | | | |
| Example 3 | Par03 | | | | | | | | |
| Example 4 | Par04 | | | | | | | | |
| Example 5 | Par05 | | | | | | | | |
| Example 6 | Par06 | | | | | | | | |
| Example 7 | Par07 | | | | | | | | |
| Example 8 | Par08 | Fir16 | 5 | | | | | | |
| Example 9 | Par09 | Fir1 | 5 | | | | | | |
| Example 10 | Par010 | Fir2 | 5 | | | | | | |
| Example 11 | Par011 | Fir3 | 5 | | | | | | |
| Example 12 | Par012 | Fir4 | 5 | | | | | | |
| Example 13 | Par013 | Fir5 | 5 | | | | | | |
| Example 14 | Par014 | Fir6 | 5 | | | | | | |
| Example 15 | Par015 | Fir7 | 5 | | | | | | |
| Example 16 | Par016 | Fir8 | 5 | | | | | | |
| Example 17 | Par017 | Fir9 | 5 | | | | | | |
| Example 18 | Par018 | Fir10 | 5 | | | | | | |
| Example 19 | Par019 | Fir11 | 5 | | | | | | |
| Example 20 | Par020 | Fir12 | 5 | | | | | | |

(continued)

| | Particle number | Coating layer formation step | | | | | | Addition step | |
| --- | --- | --- | --- | --- | --- | --- | --- | --- | --- |
| | | First-layer compound | | Second-layer compound, wax | | Second-layer compound, polyvalent metal salt | | External additive | |
| | | Species | Amount (parts) | Species | Amount (parts) | Species | Amount (parts) | Species | Amount (parts) |
| Example 21 | Par021 | Fir13 | 5 | | | | | | |
| Example 22 | Par022 | Fir14 | 5 | | | | | | |
| Example 23 | Par023 | Fir15 | 5 | | | | | | |
| Example 24 | Par024 | Fir17 | 5 | | | | | | |
| Example 25 | Par025 | Fir18 | 5 | | | | | | |
| Example 26 | Par026 | Fir16 | 7 | Sec1 | 6 | | | | |
| Example 28 | Par028 | Fir16 | 7 | Sec2 | 6 | | | | |
| Example 29 | Par029 | Fir16 | 7 | Sec3 | 6 | | | | |
| Example 30 | Par030 | Fir16 | 7 | Sec4 | 6 | | | | |
| Example 31 | Par031 | Fir16 | 7 | Sec5 | 6 | | | | |
| Example 32 | Par032 | Fir16 | 7 | Sec6 | 6 | | | | |
| Example 33 | Par033 | Fir16 | 7 | Sec7 | 6 | | | | |
| Example 34 | Par034 | Fir16 | 7 | Sec8 | 6 | | | | |
| Example 35 | Par035 | Fir16 | 7 | Sec9 | 6 | | | | |
| Example 36 | Par036 | Fir16 | 7 | Sec10 | 6 | | | | |
| Example 37 | Par037 | Fir16 | 7 | Sec11 | 6 | | | | |
| Example 38 | Par038 | Fir16 | 7 | Sec12 | 6 | | | | |
| Example 39 | Par039 | Fir16 | 7 | Sec13 | 6 | | | | |
| Example 40 | Par040 | Fir16 | 7 | Sec14 | 6 | | | | |
| Example 41 | Par041 | Fir16 | 7 | Sec15 | 6 | | | | |

Table 1-2 Particle Production Parameters

(continued)

| Table 1-2 Particle Production Parameters | | | | | | | | | |
|---|---|---|---|---|---|---|---|---|---|
| | | Coating layer formation step | | | | | | Addition step | |
| | Particle number | First-layer compound | | Second-layer compound, wax | | Second-layer compound, polyvalent metal salt | | External additive | |
| | | Species | Amount (parts) | Species | Amount (parts) | Species | Amount (parts) | Species | Amount (parts) |
| Example 42 | Par042 | Fir16 | 12 | Sec1 | 4 | | | | |
| Example 43 | Par043 | Fir16 | 7 | Sec1 | 10 | | | | |

[Table 1-3]

| Table 1-3 Particle Production Parameters | | | | | | | | | |
|---|---|---|---|---|---|---|---|---|---|
| | | Particle precursor production step | | | | | Saponification step | | |
| | Particle number | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 44 | Par044 | Cell | 50 | 3 | 4 | 10 | 17.5 | 30 | 6 |
| Example 45 | Par045 | Cell | 50 | 3 | 4 | 10 | 17.5 | 30 | 6 |
| Example 46 | Par046 | Cell | 50 | 3 | 4 | 10 | 17.5 | 30 | 6 |
| Example 47 | Par047 | Cell | 50 | 3 | 4 | 10 | 17.5 | 30 | 6 |
| Example 48 | Par048 | Cell | 50 | 3 | 4 | 10 | 17.5 | 30 | 6 |
| Example 50 | Par050 | Cell | 50 | 3 | 4 | 10 | 17.5 | 30 | 6 |
| Example 51 | Par051 | Cell | 50 | 3 | 4 | 10 | 17.5 | 30 | 6 |
| Example 52 | Par052 | Cell | 50 | 3 | 4 | 10 | 17.5 | 30 | 6 |
| Example 53 | Par053 | Cell | 50 | 3 | 4 | 10 | 17.5 | 30 | 6 |
| Example 54 | Par054 | Cell | 50 | 1.5 | 4 | 10 | 17.5 | 30 | 6 |
| Example 55 | Par055 | Cell | 50 | 1 | 4 | 10 | 17.5 | 30 | 6 |
| Example 56 | Par056 | Cell | 65 | 3 | 4 | 10 | 17.5 | 30 | 6 |
| Example 57 | Par057 | Cell | 70 | 3 | 4 | 10 | 17.5 | 30 | 6 |
| Example 58 | Par058 | Cell | 40 | 3 | 4 | 10 | 17.5 | 30 | 6 |
| Example 59 | Par059 | Cell | 35 | 3 | 4 | 10 | 17.5 | 30 | 6 |
| Example 60 | Par060 | Cell | 50 | 3 | 4 | 7 | 17.5 | 30 | 6 |
| Example 61 | Par061 | Cell | 50 | 3 | 4 | 5 | 17.5 | 30 | 6 |
| Example 64 | Par064 | Cell | 50 | 3 | 4 | 5 | 17.5 | 30 | 6 |
| Example 65 | Par065 | Cell | 50 | 3 | 4 | 5 | 17.5 | 30 | 6 |
| Example 66 | Par066 | Cell | 50 | 3 | 4 | 10 | 17.5 | 30 | 6 |
| Example 67 | Par067 | Cell | 50 | 3 | 4 | 10 | 17.5 | 30 | 6 |
| Example 68 | Par068 | Cell | 50 | 3 | 4 | 10 | 17.5 | 30 | 6 |
| Example 69 | Par069 | Cell | 50 | 3 | 4 | 10 | 17.5 | 30 | 6 |

| Table 1-3 Particle Production Parameters | | | | | | | | | |
|---|---|---|---|---|---|---|---|---|---|
| | | Particle precursor production step | | | | | Saponification step | | |
| | Particle number | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 70 | Par70 | Cell | 50 | 3 | 4 | 10 | 17.5 | 30 | 6 |
| Example 71 | Par71 | Cell | 50 | 3 | 4 | 10 | 17.5 | 30 | 6 |
| Example 72 | Par72 | Cell | 50 | 3 | 4 | 10 | 17.5 | 30 | 6 |
| Example 73 | Par73 | Cell | 50 | 3 | 4 | 10 | 17.5 | 30 | 6 |
| Example 74 | Par74 | Cell | 50 | 3 | 4 | 10 | 17.5 | 30 | 6 |
| Example 75 | Par75 | Cell | 50 | 3 | 4 | 10 | 17.5 | 30 | 6 |
| Example 76 | Par76 | Cell | 50 | 3 | 4 | 10 | 17.5 | 30 | 6 |
| Example 77 | Par77 | Cell | 50 | 3 | 4 | 10 | 17.5 | 30 | 6 |
| Example 78 | Par78 | Cell | 50 | 3 | 4 | 10 | 17.5 | 30 | 6 |
| Example 79 | Par79 | Cell | 50 | 3 | 4 | 10 | 17.5 | 30 | 6 |
| Example 80 | Par80 | Cell | 50 | 3 | 4 | 10 | 17.5 | 30 | 6 |
| Example 81 | Par81 | Cell | 50 | 3 | 4 | 10 | 17.5 | 30 | 6 |
| Example 82 | Par82 | Cell | 50 | 3 | 4 | 10 | 17.5 | 30 | 6 |
| Example 83 | Par83 | Cell | 50 | 3 | 6 | 10 | 15 | 30 | 2 |
| Example 84 | Par84 | Cell | 50 | 3 | 8 | 10 | 15 | 30 | 2 |

EP 4 223 273 B1

[Table 1-4]

| Table 1-4 Particle Production Parameters | | | | | | | | | |
|---|---|---|---|---|---|---|---|---|---|
| | | Coating layer formation step | | | | | | Addition step | |
| | Particle number | First-layer compound | | Second-layer compound, wax | | Second-layer compound, polyvalent metal salt | | External additive | |
| | | Species | Amount (parts) | Species | Amount (parts) | Species | Amount (parts) | Species | Amount (parts) |
| Example 44 | Par044 | Fir16 | 12 | Sec1 | 10 | | | | |
| Example 45 | Par045 | Fir16 | 7 | Sec1 | 6 | | | Surl | 0.6 |
| Example 46 | Par046 | Fir16 | 7 | Sec1 | 6 | | | Sur2 | 0.6 |
| Example 47 | Par047 | Fir16 | 7 | Sec1 | 6 | | | Sur3 | 0.6 |
| Example 48 | Par048 | Fir16 | 7 | Sec1 | 6 | | | Sur4 | 0.6 |
| Example 50 | Par050 | Fir16 | 7 | Sec1 | 6 | | | Sur6 | 0.6 |
| Example 51 | Par051 | Fir16 | 7 | Sec1 | 6 | | | Sur7 | 0.6 |
| Example 52 | Par052 | Fir16 | 7 | Sec1 | 6 | | | Surl | 0.3 |
| Example 53 | Par053 | Fir16 | 7 | Sec1 | 6 | | | Surl | 0.9 |
| Example 54 | Par054 | Fir16 | 7 | Sec1 | 6 | | | | |
| Example 55 | Par055 | Fir16 | 7 | Sec1 | 6 | | | | |
| Example 56 | Par056 | Fir16 | 7 | Sec1 | 6 | | | | |
| Example 57 | Par057 | Fir16 | 7 | Sec1 | 6 | | | | |
| Example 58 | Par058 | Fir16 | 7 | Sec1 | 6 | | | | |
| Example 59 | Par059 | Fir16 | 7 | Sec1 | 6 | | | | |
| Example 60 | Par060 | Fir16 | 7 | Sec1 | 6 | | | | |
| Example 61 | Par061 | Fir16 | 7 | Sec1 | 6 | | | | |
| Example 64 | Par064 | | | Sec1 | 6 | | | | |
| Example 65 | Par065 | | | Sec1 | 6 | | | Surl | 0.6 |
| Example 66 | Par066 | Fir16 | 7 | Sec3 | 6 | Sec21 | 0.03 | Surl | 0.6 |

(continued)

| | Particle number | Coating layer formation step | | | | | | Addition step | |
|---|---|---|---|---|---|---|---|---|---|
| Table 1-4 Particle Production Parameters | | | | | | | | | |
| | | First-layer compound | | Second-layer compound, wax | | Second-layer compound, polyvalent metal salt | | External additive | |
| | | Species | Amount (parts) | Species | Amount (parts) | Species | Amount (parts) | Species | Amount (parts) |
| Example 67 | Par067 | Fir16 | 7 | Sec1 | 6 | Sec22 | 0.03 | Surl | 0.6 |
| Example 68 | Par068 | Fir16 | 7 | Sec1 | 6 | Sec23 | 0.03 | Surl | 0.6 |
| Example 69 | Par069 | Fir16 | 7 | Sec1 | 6 | Sec24 | 0.03 | Surl | 0.6 |
| Example 70 | Par70 | Fir19 | 8 | | | | | | |
| Example 71 | Par71 | Fir20 | 8 | | | | | | |
| Example 72 | Par72 | Fir21 | 8 | | | | | | |
| Example 73 | Par73 | Fir22 | 8 | | | | | | |
| Example 74 | Par74 | Fir23 | 8 | | | | | | |
| Example 75 | Par75 | Fir24 | 8 | | | | | | |
| Example 76 | Par76 | Fir25 | 8 | | | | | | |
| Example 77 | Par77 | Fir26 | 8 | | | | | | |
| Example 78 | Par78 | Fir19 | 6 | | | | | | |
| Example 79 | Par79 | Fir19 | 10 | | | | | | |
| Example 80 | Par80 | Fir19 | 8 | Sec1 | 4 | | | | |
| Example 81 | Par81 | Fir19 | 8 | Sec1 | 4 | Sec21 | 0.012 | | |
| Example 82 | Par82 | Fir19 | 8 | Sec1 | 4 | Sec21 | 0.012 | Surl | 0.6 |
| Example 83 | Par83 | Fir16 | 7 | Sec1 | 6 | | | Surl | 0.6 |
| Example 84 | Par84 | Fir16 | 7 | Sec1 | 6 | | | Surl | 0.6 |

[Table 1-5]

Table 1-5 Particle Production Parameters

| Class | Particle number | Particle precursor production step | | | | | Saponification step | | |
|---|---|---|---|---|---|---|---|---|---|
| | | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 101 | Par701 | Cel2 | 50 | 3 | 4 | 10 | 17.5 | 30 | 6 |
| Example 102 | Par702 | Cel2 | 50 | 3 | 4 | 10 | 17.5 | 30 | 6 |
| Example 103 | Par703 | Cel2 | 50 | 3 | 4 | 10 | 17.5 | 30 | 6 |
| Example 104 | Par704 | Cel2 | 50 | 3 | 4 | 10 | 17.5 | 30 | 6 |
| Example 105 | Par705 | Cel2 | 50 | 3 | 4 | 10 | 17.5 | 30 | 6 |
| Example 106 | Par706 | Cel2 | 50 | 3 | 4 | 10 | 17.5 | 30 | 6 |
| Example 107 | Par707 | Cel2 | 50 | 3 | 4 | 10 | 17.5 | 30 | 6 |
| Example 108 | Par708 | Cel2 | 50 | 3 | 4 | 10 | 17.5 | 30 | 6 |
| Example 109 | Par709 | Cel2 | 50 | 3 | 4 | 10 | 17.5 | 30 | 6 |
| Example 110 | Par710 | Cel2 | 50 | 3 | 4 | 10 | 17.5 | 30 | 6 |
| Example 111 | Par711 | Cel2 | 50 | 3 | 4 | 10 | 17.5 | 30 | 6 |
| Example 112 | Par712 | Cel2 | 50 | 3 | 4 | 10 | 17.5 | 30 | 6 |
| Example 113 | Par713 | Cel2 | 50 | 3 | 4 | 10 | 17.5 | 30 | 6 |
| Example 114 | Par714 | Cel2 | 50 | 3 | 4 | 10 | 17.5 | 30 | 6 |
| Example 115 | Par715 | Cel2 | 50 | 3 | 4 | 10 | 17.5 | 30 | 6 |
| Example 116 | Par716 | Cel2 | 50 | 3 | 4 | 10 | 17.5 | 30 | 6 |
| Example 117 | Par717 | Cel2 | 50 | 3 | 4 | 10 | 17.5 | 30 | 6 |
| Example 118 | Par718 | Cel2 | 50 | 3 | 4 | 10 | 17.5 | 30 | 6 |
| Example 119 | Par719 | Cel2 | 50 | 3 | 4 | 10 | 17.5 | 30 | 6 |
| Example 120 | Par720 | Cel2 | 50 | 3 | 4 | 10 | 17.5 | 30 | 6 |
| Example 121 | Par721 | Cel2 | 50 | 3 | 4 | 10 | 17.5 | 30 | 6 |
| Example 122 | Par722 | Cel2 | 50 | 3 | 4 | 10 | 17.5 | 30 | 6 |
| Example 123 | Par723 | Cel2 | 50 | 3 | 4 | 10 | 17.5 | 30 | 6 |

| Table 1-5 Particle Production Parameters | | | | | | | | | |
|---|---|---|---|---|---|---|---|---|---|
| | | Particle precursor production step | | | | | Saponification step | | |
| Class | Particle number | Resin species | Amount of calcium carbonate (parts) | First stirring time (hr) | Amount of CMC (parts) | Amount of sodium hydroxide (g) | Amount of 20% NaOHaq (parts) | Saponification temperature (°C) | Duration of stirring (hr) |
| Example 124 | Par724 | Cel2 | 50 | 3 | 4 | 10 | 17.5 | 30 | 6 |

[Table 1-6]

| Table 1-6 Particle Production Parameters | | | | | | | |
|---|---|---|---|---|---|---|---|
| | | Coating layer formation step | | | | Addition step | |
| Class | Particle number | First-layer compound | | Second-layer compound | | External additive | |
| | | Species | Amount (parts) | Species | Amount (parts) | Species | Amount (parts) |
| Example 101 | Par701 | | | Fir41 | 8 | | |
| Example 102 | Par702 | | | Sec1 | 8 | | |
| Example 103 | Par703 | | | Sec15 | 8 | | |
| Example 104 | Par704 | Fir31 | 1 | Fir19 | 8 | | |
| Example 105 | Par705 | Fir31 | 1 | Fir26 | 8 | | |
| Example 106 | Par706 | Fir32 | 1 | Fir41 | 8 | | |
| Example 107 | Par707 | Fir32 | 1 | Fir19 | 8 | | |
| Example 108 | Par708 | Fir32 | 1 | Fir26 | 8 | | |
| Example 109 | Par709 | Fir32 | 1 | Fir41 | 8 | | |
| Example 110 | Par710 | Fir33 | 1 | Fir19 | 8 | | |
| Example 111 | Par711 | Fir33 | 1 | Fir26 | 8 | | |
| Example 112 | Par712 | Fir33 | 1 | Fir41 | 8 | | |
| Example 113 | Par713 | Fir34 | 1 | Fir19 | 8 | | |
| Example 114 | Par714 | Fir34 | 1 | Fir26 | 8 | | |
| Example 115 | Par715 | Fir34 | 1 | Fir41 | 8 | | |
| Example 116 | Par716 | Fir16 | 1 | Fir19 | 8 | | |
| Example 117 | Par717 | Fir16 | 1 | Fir26 | 8 | | |
| Example 118 | Par718 | Fir16 | 1 | Fir41 | | | |
| Example 119 | Par719 | Fir31 | 1 | Sec1 | 8 | | |
| Example 120 | Par720 | Fir32 | 1 | Sec1 | 8 | | |
| Example 121 | Par721 | Fir33 | 1 | Sec1 | 8 | | |
| Example 122 | Par722 | Fir34 | 1 | Sec1 | 8 | | |
| Example 123 | Par723 | Fir32 | 1 | Fir41 | 8 | Surl | 0.6 |
| Example 124 | Par724 | Fir32 | 1 | Fir41 | 8 | Sur2 | 0.6 |

[Table 2-1]

| Table 2-1 Evaluation Results | | | | | | | | |
|---|---|---|---|---|---|---|---|---|
| | Particle number | Particle characteristics | | | | | | |
| | | Biodegradation, 5 days (%) | Biodegradation, 60 days (%) | Particle diameter ($\mu$m) | GSDv | Sphericity (-) | Mn (-) | Surface smoothness (%) |
| Example 1 | Par01 | 16 | 97 | 8 | 1.13 | 0.98 | 46000 | 93 |
| Example 2 | Par02 | 14 | 97 | 7 | 1.25 | 0.96 | 59000 | 94 |
| Example 3 | Par03 | 12 | 94 | 8 | 1.38 | 0.95 | 73000 | 95 |

(continued)

| Table 2-1 Evaluation Results | | | | | | | | |
|---|---|---|---|---|---|---|---|---|
| | | Particle characteristics | | | | | | |
| | Particle number | Biodegradation, 5 days (%) | Biodegradation, 60 days (%) | Particle diameter ($\mu$m) | GSDv | Sphericity (-) | Mn (-) | Surface smoothness (%) |
| Example 4 | Par04 | 18 | 93 | 6 | 1.44 | 0.96 | 49000 | 95 |
| Example 5 | Par05 | 18 | 78 | 8 | 1.38 | 0.94 | 36000 | 89 |
| Example 6 | Par06 | 18 | 78 | 7 | 1.39 | 0.95 | 23000 | 88 |
| Example 7 | Par07 | 15 | 82 | 6 | 1.28 | 0.98 | 12000 | 87 |
| Example 8 | Par08 | 17 | 88 | 7 | 1.23 | 0.99 | 47000 | 95 |
| Example 9 | Par09 | 15 | 80 | 8 | 1.28 | 0.95 | 45000 | 93 |
| Example 10 | Par010 | 14 | 77 | 7 | 1.31 | 0.98 | 48000 | 93 |
| Example 11 | Par011 | 13 | 83 | 6 | 1.29 | 0.97 | 46000 | 94 |
| Example 12 | Par012 | 11 | 80 | 8 | 1.33 | 0.96 | 43000 | 92 |
| Example 13 | Par013 | 16 | 83 | 7 | 1.34 | 0.96 | 47000 | 93 |
| Example 14 | Par014 | 12 | 81 | 6 | 1.28 | 0.98 | 47000 | 94 |
| Example 15 | Par015 | 15 | 81 | 7 | 1.31 | 0.97 | 46000 | 92 |
| Example 16 | Par016 | 17 | 86 | 8 | 1.27 | 0.96 | 47000 | 92 |
| Example 17 | Par017 | 18 | 77 | 6 | 1.29 | 0.95 | 48000 | 90 |
| Example 18 | Par018 | 17 | 82 | 7 | 1.35 | 0.98 | 47000 | 92 |
| Example 19 | Par019 | 15 | 77 | 8 | 1.28 | 0.97 | 47000 | 91 |
| Example 20 | Par020 | 14 | 78 | 7 | 1.33 | 0.95 | 45000 | 93 |
| Example 21 | Par021 | 14 | 80 | 6 | 1.45 | 0.96 | 47000 | 92 |
| Example 22 | Par022 | 13 | 81 | 8 | 1.38 | 0.97 | 45000 | 93 |
| Example 23 | Par023 | 11 | 82 | 7 | 1.35 | 0.96 | 47000 | 92 |
| Example 24 | Par024 | 14 | 64 | 6 | 1.36 | 0.98 | 47000 | 88 |

(continued)

| | Particle number | Particle characteristics | | | | | | |
|---|---|---|---|---|---|---|---|---|
| | | Biodegradation, 5 days (%) | Biodegradation, 60 days (%) | Particle diameter (μm) | GSDv | Sphericity (-) | Mn (-) | Surface smoothness (%) |
| Example 25 | Par025 | 14 | 63 | 8 | 1.41 | 0.97 | 48000 | 89 |
| Example 26 | Par026 | 17 | 80 | 8 | 1.12 | 0.98 | 46000 | 89 |
| Example 28 | Par028 | 16 | 81 | 7 | 1.38 | 0.98 | 47000 | 88 |
| Example 29 | Par029 | 15 | 80 | 7 | 1.36 | 0.96 | 45000 | 87 |
| Example 30 | Par030 | 13 | 77 | 8 | 1.36 | 0.98 | 47000 | 87 |
| Example 31 | Par031 | 17 | 79 | 7 | 1.38 | 0.98 | 47000 | 88 |
| Example 32 | Par032 | 11 | 78 | 8 | 1.39 | 0.96 | 45000 | 87 |
| Example 33 | Par033 | 15 | 80 | 7 | 1.37 | 0.98 | 47000 | 85 |
| Example 34 | Par034 | 12 | 77 | 6 | 1.41 | 0.96 | 46000 | 85 |
| Example 35 | Par035 | 11 | 75 | 7 | 1.38 | 0.98 | 47000 | 83 |
| Example 36 | Par036 | 12 | 77 | 8 | 1.35 | 0.98 | 47000 | 85 |
| Example 37 | Par037 | 13 | 76 | 8 | 1.33 | 0.98 | 47000 | 80 |
| Example 38 | Par038 | 14 | 77 | 7 | 1.36 | 0.97 | 48000 | 83 |
| Example 39 | Par039 | 13 | 75 | 6 | 1.38 | 0.98 | 47000 | 81 |
| Example 40 | Par040 | 15 | 78 | 7 | 1.39 | 0.96 | 45000 | 82 |
| Example 41 | Par041 | 12 | 66 | 8 | 1.33 | 0.98 | 47000 | 83 |
| Example 42 | Par042 | 13 | 76 | 7 | 1.43 | 0.99 | 47000 | 84 |

Table 2-1 Evaluation Results

[Table 2-2]

| Table 2-2 Evaluation Results | | | Smoothness | | | Moist sensation | | | Softness | | |
|---|---|---|---|---|---|---|---|---|---|---|---|
| | Particle number | | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | | Initial | 96 hours | Change | Initial | 96 hours | Change | Initial | 96 hours | Change |
| Example 1 | Par01 | | 9 | 6 | 3 | 9 | 6 | 3 | 9 | 6 | 3 |
| Example 2 | Par02 | | 9 | 6 | 3 | 9 | 6 | 3 | 9 | 6 | 3 |
| Example 3 | Par03 | | 8 | 5 | 3 | 8 | 5 | 3 | 7 | 4 | 3 |
| Example 4 | Par04 | | 8 | 5 | 3 | 8 | 5 | 3 | 7 | 4 | 2 |
| Example 5 | Par05 | | 8 | 4 | 4 | 8 | 6 | 2 | 7 | 4 | 3 |
| Example 6 | Par06 | | 8 | 4 | 4 | 7 | 5 | 2 | 7 | 4 | 3 |
| Example 7 | Par07 | | 8 | 4 | 4 | 8 | 6 | 2 | 7 | 4 | 3 |
| Example 8 | Par08 | | 9 | 7 | 2 | 9 | 7 | 2 | 8 | 6 | 2 |
| Example 9 | Par09 | | 8 | 6 | 2 | 8 | 6 | 2 | 7 | 5 | 2 |
| Example 10 | Par010 | | 7 | 5 | 2 | 8 | 6 | 2 | 7 | 5 | 2 |
| Example 11 | Par011 | | 8 | 6 | 2 | 8 | 6 | 2 | 7 | 5 | 2 |
| Example 12 | Par012 | | 8 | 6 | 2 | 8 | 6 | 2 | 8 | 6 | 2 |
| Example 13 | Par013 | | 8 | 6 | 2 | 8 | 6 | 2 | 7 | 5 | 2 |
| Example 14 | Par014 | | 7 | 5 | 2 | 8 | 6 | 2 | 7 | 5 | 2 |
| Example 15 | Par015 | | 8 | 6 | 2 | 8 | 6 | 2 | 7 | 5 | 2 |
| Example 16 | Par016 | | 8 | 6 | 2 | 8 | 6 | 2 | 8 | 6 | 2 |
| Example 17 | Par017 | | 7 | 5 | 2 | 8 | 6 | 2 | 8 | 6 | 2 |
| Example 18 | Par018 | | 8 | 6 | 2 | 8 | 6 | 2 | 7 | 5 | 2 |
| Example 19 | Par019 | | 7 | 5 | 2 | 8 | 6 | 2 | 8 | 6 | 2 |
| Example 20 | Par020 | | 8 | 6 | 2 | 8 | 6 | 2 | 7 | 5 | 2 |

(continued)

| Table 2-2 Evaluation Results | | | | | | | | | | |
|---|---|---|---|---|---|---|---|---|---|---|
| | | Smoothness | | | Moist sensation | | | Softness | | |
| | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 21 | Par021 | 8 | 6 | 2 | 8 | 6 | 2 | 7 | 5 | 2 |
| Example 22 | Par022 | 8 | 6 | 2 | 8 | 6 | 2 | 7 | 5 | 2 |
| Example 23 | Par023 | 8 | 6 | 2 | 8 | 6 | 2 | 7 | 5 | 2 |
| Example 24 | Par024 | 7 | 4 | 3 | 8 | 5 | 3 | 8 | 4 | 4 |
| Example 25 | Par025 | 8 | 6 | 2 | 8 | 4 | 4 | 7 | 5 | 2 |
| Example 26 | Par026 | 10 | 8 | 2 | 9 | 7 | 2 | 10 | 8 | 2 |
| Example 28 | Par028 | 10 | 8 | 2 | 9 | 7 | 2 | 9 | 7 | 2 |
| Example 29 | Par029 | 10 | 8 | 2 | 9 | 7 | 2 | 9 | 7 | 2 |
| Example 30 | Par030 | 10 | 8 | 2 | 9 | 7 | 2 | 9 | 7 | 2 |
| Example 31 | Par031 | 10 | 8 | 2 | 9 | 7 | 2 | 9 | 7 | 2 |
| Example 32 | Par032 | 10 | 8 | 2 | 9 | 7 | 2 | 9 | 7 | 2 |
| Example 33 | Par033 | 10 | 8 | 2 | 9 | 7 | 2 | 9 | 7 | 2 |
| Example 34 | Par034 | 9 | 7 | 2 | 9 | 7 | 2 | 9 | 7 | 2 |
| Example 35 | Par035 | 9 | 7 | 2 | 9 | 7 | 2 | 9 | 7 | 2 |
| Example 36 | Par036 | 9 | 7 | 2 | 9 | 7 | 2 | 9 | 7 | 2 |
| Example 37 | Par037 | 9 | 7 | 2 | 9 | 7 | 2 | 9 | 7 | 2 |
| Example 38 | Par038 | 9 | 7 | 2 | 9 | 7 | 2 | 9 | 7 | 2 |
| Example 39 | Par039 | 9 | 7 | 2 | 9 | 7 | 2 | 9 | 7 | 2 |
| Example 40 | Par040 | 9 | 7 | 2 | 9 | 7 | 2 | 9 | 7 | 2 |
| Example 41 | Par041 | 9 | 7 | 2 | 9 | 7 | 2 | 8 | 6 | 2 |

(continued)

| Table 2-2 Evaluation Results | | | | | | | | | | |
|---|---|---|---|---|---|---|---|---|---|---|
| | | Smoothness | | | Moist sensation | | | Softness | | |
| | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 42 | Par042 | 10 | 8 | 2 | 9 | 7 | 2 | 10 | 8 | 2 |

[Table 2-3]

| Table 2-3 Evaluation Results | | | | | | | | |
|---|---|---|---|---|---|---|---|---|
| | | Particle characteristics | | | | | | |
| | Particle number | Biodegradation, 5 days (%) | Biodegradation, 60 days (%) | Particle diameter (μm) | GSDv (-) | Sphericity (-) | Mn (-) | Surface smoothness (%) |
| Example 43 | Par043 | 14 | 78 | 7 | 1.36 | 0.98 | 44000 | 80 |
| Example 44 | Par044 | 12 | 72 | 8 | 1.35 | 0.96 | 48000 | 78 |
| Example 45 | Par045 | 11 | 76 | 6 | 1.14 | 0.98 | 47000 | 80 |
| Example 46 | Par046 | 10 | 76 | 8 | 1.33 | 0.99 | 45000 | 80 |
| Example 47 | Par047 | 9 | 75 | 7 | 1.32 | 0.96 | 47000 | 80 |
| Example 48 | Par048 | 10 | 75 | 8 | 1.38 | 0.96 | 47000 | 81 |
| Example 50 | Par050 | 9 | 63 | 7 | 1.32 | 0.98 | 47000 | 82 |
| Example 51 | Par051 | 10 | 62 | 8 | 1.33 | 0.98 | 45000 | 81 |
| Example 52 | Par052 | 11 | 76 | 7 | 1.45 | 0.97 | 4800 | 81 |
| Example 53 | Par053 | 7 | 76 | 8 | 1.27 | 0.98 | 47000 | 80 |
| Example 54 | Par054 | 13 | 78 | 7 | 1.69 | 0.98 | 47000 | 83 |
| Example 55 | Par055 | 12 | 68 | 8 | 1.74 | 0.97 | 46000 | 88 |
| Example 56 | Par056 | 14 | 78 | 3 | 1.44 | 0.98 | 47000 | 87 |
| Example 57 | Par057 | 14 | 66 | 2 | 1.45 | 0.98 | 47000 | 88 |
| Example 58 | Par058 | 13 | 79 | 9 | 1.38 | 0.97 | 47000 | 89 |

(continued)

| Table 2-3 Evaluation Results | | | | | | | |
|---|---|---|---|---|---|---|---|
| | | Particle characteristics | | | | | |
| | Particle number | Biodegradation, 5 days (%) | Biodegradation, 60 days (%) | Particle diameter (μm) | GSDv (-) | Sphericity (-) | Mn (-) | Surface smoothness (%) |
| Example 59 | Par059 | 15 | 65 | 11 | 1.31 | 0.98 | 45000 | 90 |
| Example 60 | Par060 | 13 | 78 | 8 | 1.33 | 0.91 | 47000 | 88 |
| Example 61 | Par061 | 12 | 68 | 7 | 1.35 | 0.85 | 47000 | 87 |
| Example 64 | Par064 | 15 | 90 | 8 | 1.38 | 0.96 | 45000 | 81 |
| Example 65 | Par065 | 12 | 87 | 7 | 1.39 | 0.97 | 47000 | 82 |
| Example 66 | Par066 | 7 | 70 | 7 | 1.32 | 0.98 | 47000 | 83 |
| Example 67 | Par067 | 6 | 70 | 8 | 1.33 | 0.98 | 47000 | 83 |
| Example 68 | Par068 | 8 | 70 | 8 | 1.38 | 0.98 | 47000 | 83 |
| Example 69 | Par069 | 7 | 70 | 7 | 1.35 | 0.98 | 47000 | 84 |
| Example 70 | Par70 | 10 | 95 | 7 | 1.38 | 0.97 | 46000 | 95 |
| Example 71 | Par71 | 15 | 93 | 6 | 1.33 | 0.96 | 45000 | 95 |
| Example 72 | Par72 | 15 | 92 | 8 | 1.41 | 0.97 | 45000 | 96 |
| Example 73 | Par73 | 18 | 92 | 6 | 1.43 | 0.95 | 46000 | 95 |
| Example 74 | Par74 | 9 | 79 | 7 | 1.45 | 0.93 | 45000 | 93 |
| Example 75 | Par75 | 13 | 95 | 7 | 1.38 | 0.97 | 45000 | 94 |
| Example 76 | Par76 | 12 | 95 | 6 | 1.36 | 0.94 | 46000 | 95 |
| Example 77 | Par77 | 13 | 72 | 8 | 1.41 | 0.93 | 45000 | 95 |
| Example 78 | Par78 | 10 | 95 | 7 | 1.38 | 0.95 | 46000 | 95 |
| Example 79 | Par79 | 9 | 95 | 6 | 1.44 | 0.96 | 45000 | 96 |
| Example 80 | Par80 | 10 | 80 | 8 | 1.37 | 0.95 | 46000 | 95 |
| Example 81 | Par81 | 8 | 80 | 7 | 1.35 | 0.9 | 45000 | 85 |

(continued)

| Table 2-3 Evaluation Results | | | | | | | | |
|---|---|---|---|---|---|---|---|---|
| | Particle number | Particle characteristics | | | | | | |
| | | Biodegradation, 5 days (%) | Biodegradation, 60 days (%) | Particle diameter (μm) | GSDv (-) | Sphericity (-) | Mn (-) | Surface smoothness (%) |
| Example 82 | Par82 | 7 | 79 | 6 | 1.36 | 0.95 | 46000 | 86 |
| Example 83 | Par83 | 16 | 78 | 7 | 1.44 | 0.94 | 46000 | 82 |
| Example 84 | Par84 | 12 | 68 | 8 | 1.47 | 0.91 | 46000 | 78 |

[Table 2-4]

| Table 2-4 Evaluation Results | | | | | | | | | | |
|---|---|---|---|---|---|---|---|---|---|---|
| | Particle number | Smoothness | | | Moist sensation | | | Softness | | |
| | | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 43 | Par043 | 10 | 8 | 2 | 9 | 7 | 2 | 10 | 8 | 2 |
| Example 44 | Par044 | 9 | 7 | 2 | 9 | 7 | 2 | 9 | 6 | 3 |
| Example 45 | Par045 | 10 | 9 | 1 | 10 | 8 | 2 | 10 | 9 | 1 |
| Example 46 | Par046 | 10 | 9 | 1 | 10 | 8 | 2 | 10 | 9 | 1 |
| Example 47 | Par047 | 10 | 9 | 1 | 9 | 7 | 2 | 10 | 9 | 1 |
| Example 48 | Par048 | 10 | 9 | 1 | 9 | 7 | 2 | 10 | 9 | 1 |
| Example 50 | Par050 | 10 | 9 | 1 | 9 | 7 | 2 | 10 | 8 | 2 |
| Example 51 | Par051 | 10 | 9 | 1 | 9 | 7 | 2 | 10 | 8 | 2 |
| Example 52 | Par052 | 10 | 9 | 1 | 10 | 8 | 2 | 10 | 9 | 1 |
| Example 53 | Par053 | 10 | 9 | 1 | 10 | 8 | 2 | 10 | 9 | 1 |
| Example 54 | Par054 | 10 | 8 | 2 | 9 | 7 | 2 | 10 | 8 | 2 |
| Example 55 | Par055 | 9 | 7 | 2 | 9 | 7 | 2 | 9 | 6 | 3 |
| Example 56 | Par056 | 10 | 8 | 2 | 9 | 7 | 2 | 10 | 8 | 2 |

(continued)

| | | Smoothness | | | Moist sensation | | | Softness | | |
|---|---|---|---|---|---|---|---|---|---|---|
| | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 57 | Par057 | 9 | 7 | 2 | 9 | 7 | 2 | 8 | 5 | 3 |
| Example 58 | Par058 | 10 | 8 | 2 | 9 | 7 | 2 | 10 | 8 | 2 |
| Example 59 | Par059 | 8 | 6 | 2 | 9 | 7 | 2 | 8 | 5 | 3 |
| Example 60 | Par060 | 10 | 8 | 2 | 9 | 7 | 2 | 10 | 8 | 2 |
| Example 61 | Par061 | 9 | 7 | 1 | 9 | 7 | 2 | 10 | 7 | 3 |
| Example 64 | Par064 | 9 | 6 | 3 | 8 | 6 | 2 | 8 | 6 | 3 |
| Example 65 | Par065 | 9 | 7 | 2 | 8 | 7 | 1 | 8 | 7 | 1 |
| Example 66 | Par066 | 10 | 10 | 0 | 10 | 10 | 0 | 10 | 10 | 0 |
| Example 67 | Par067 | 10 | 10 | 0 | 10 | 10 | 0 | 10 | 10 | 0 |
| Example 68 | Par068 | 10 | 10 | 0 | 10 | 10 | 0 | 10 | 10 | 0 |
| Example 69 | Par069 | 10 | 10 | 0 | 10 | 10 | 0 | 10 | 10 | 0 |
| Example 70 | Par70 | 10 | 8 | 2 | 9 | 7 | 2 | 10 | 8 | 2 |
| Example 71 | Par71 | 9 | 8 | 1 | 9 | 8 | 1 | 9 | 8 | 1 |
| Example 72 | Par72 | 9 | 8 | 1 | 9 | 8 | 1 | 9 | 7 | 2 |
| Example 73 | Par73 | 9 | 7 | 2 | 9 | 7 | 2 | 9 | 7 | 2 |
| Example 74 | Par74 | 9 | 8 | 1 | 9 | 7 | 2 | 9 | 7 | 2 |
| Example 75 | Par75 | 10 | 8 | 2 | 9 | 7 | 2 | 10 | 8 | 2 |
| Example 76 | Par76 | 10 | 8 | 2 | 9 | 7 | 2 | 10 | 8 | 2 |
| Example 77 | Par77 | 10 | 8 | 2 | 9 | 7 | 2 | 10 | 8 | 2 |
| Example 78 | Par78 | 10 | 8 | 2 | 9 | 7 | 2 | 10 | 8 | 2 |

Table 2-4 Evaluation Results

(continued)

| Table 2-4 Evaluation Results | | | | | | | | | | | |
|---|---|---|---|---|---|---|---|---|---|---|---|
| | | Smoothness | | | Moist sensation | | | Softness | | | |
| | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | | |
| | | Initial | 96 hours | Change | Initial | 96 hours | Change | Initial | 96 hours | Change |
| Example 79 | Par79 | 10 | 9 | 1 | 9 | 7 | 2 | 10 | 9 | 1 |
| Example 80 | Par80 | 10 | 9 | 1 | 9 | 7 | 2 | 10 | 9 | 1 |
| Example 81 | Par81 | 10 | 9 | 1 | 10 | 8 | 2 | 10 | 9 | 1 |
| Example 82 | Par82 | 10 | 10 | 0 | 10 | 8 | 2 | 10 | 10 | 0 |
| Example 83 | Par83 | 10 | 9 | 1 | 10 | 8 | 2 | 10 | 9 | 1 |
| Example 84 | Par84 | 9 | 8 | 1 | 10 | 8 | 2 | 9 | 7 | 2 |

[Table 2-5]

Table 2-5 Evaluation Results

| | Particle number | Particle characteristics | | | | | | |
|---|---|---|---|---|---|---|---|---|
| | | Biodegradation, 5 days (%) | Biodegradation, 60 days (%) | Particle diameter (μm) | GSDv (-) | Sphericity (-) | Mn (-) | Surface smoothness (%) |
| Comparative Example 1 | Par101 | 38 | 79 | 14 | 1.17 | 0.97 | 110000 | 98 |
| Comparative Example 2 | Par102 | 1 | 25 | 14 | 1.32 | 0.98 | 110000 | 90 |
| Comparative Example 3 | Par103 | 2 | 24 | 12 | 1.47 | 0.55 | 110000 | 45 |
| Comparative Example 4 | Par104 | 30 | 78 | 10 | 1.86 | 0.97 | 45000 | 90 |
| Comparative Example 5 | Par111 | 49 | 80 | 10 | 1.67 | 0.96 | 21000 | 82 |
| Comparative Example 6 | Par112 | 48 | 80 | 12.7 | 1.72 | 0.96 | 12000 | 79 |
| Comparative Example 7 | Par113 | 33 | 78 | 4 | 1.87 | 0.95 | 44000 | 90 |
| Comparative Example 8 | Par114 | 30 | 79 | 8.2 | 1.88 | 0.96 | 45000 | 90 |
| Comparative Example 9 | Par115 | 1 | 17 | 12 | 1.94 | 0.98 | 48000 | 88 |
| Comparative Example 10 | Par116 | 49 | 85 | 9 | 1.45 | 0.96 | 33000 | 92 |
| Comparative Example 11 | Par117 | 48 | 88 | 9 | 1.55 | 0.96 | 32000 | 92 |

EP 4 223 273 B1

[Table 2-6]

Table 2-6 Evaluation Results

| | Particle number | Smoothness | | | Moist sensation | | | Softness | | |
|---|---|---|---|---|---|---|---|---|---|---|
| | | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Comparative Example 1 | Par101 | 5 | 1 | 4 | 4 | 1 | 3 | 4 | 1 | 3 |
| Comparative Example 2 | Par102 | 9 | 4 | 5 | 8 | 4 | 4 | 8 | 5 | 3 |
| Comparative Example 3 | Par103 | 8 | 3 | 5 | 8 | 3 | 5 | 8 | 3 | 5 |
| Comparative Example 4 | Par104 | 8 | 3 | 5 | 8 | 3 | 5 | 8 | 3 | 5 |
| Comparative Example 5 | Par111 | 8 | 3 | 5 | 8 | 3 | 5 | 8 | 3 | 5 |
| Comparative Example 6 | Par112 | 7 | 2 | 5 | 8 | 3 | 5 | 7 | 3 | 4 |
| Comparative Example 7 | Par113 | 6 | 2 | 4 | 7 | 2 | 5 | 6 | 2 | 4 |
| Comparative Example 8 | Par114 | 7 | 2 | 5 | 7 | 2 | 5 | 7 | 4 | 3 |
| Comparative Example 9 | Par115 | 7 | 2 | 5 | 7 | 2 | 5 | 8 | 3 | 5 |
| Comparative Example 10 | Par116 | 8 | 3 | 5 | 7 | 2 | 5 | 7 | 2 | 5 |
| Comparative Example 11 | Par117 | 7 | 3 | 4 | 7 | 3 | 4 | 7 | 2 | 5 |

[Table 2-7]

Table 2-7 Evaluation Results

| Class | Particle number | Particle characteristics | | | | | | |
|---|---|---|---|---|---|---|---|---|
| | | Biodegradation, 5 days (%) | Biodegradation, 60 days (%) | Particle diameter ($\mu$m) | GSD (-) | Sphericity (-) | Mn (-) | Surface smoothness (%) |
| Example 101 | Par701 | 5 | 95 | 8 | 1.34 | 0.94 | 46000 | 90 |
| Example 102 | Par702 | 11 | 94 | 7 | 1.45 | 0.93 | 46000 | 89 |
| Example 103 | Par703 | 15 | 66 | 9 | 1.44 | 0.95 | 47000 | 81 |
| Example 104 | Par704 | 9 | 92 | 8 | 1.35 | 0.94 | 46000 | 87 |
| Example 105 | Par705 | 13 | 93 | 9 | 1.38 | 0.94 | 47000 | 88 |

(continued)

| | | Particle characteristics | | | | | | |
|---|---|---|---|---|---|---|---|---|
| Class | Particle number | Biodegradation, 5 days (%) | Biodegradation, 60 days (%) | Particle diameter (μm) | GSD (-) | Sphericity (-) | Mn (-) | Surface smoothness (%) |
| Example 106 | Par706 | 3 | 92 | 7 | 1.34 | 0.95 | 45000 | 89 |
| Example 107 | Par707 | 8 | 91 | 6 | 1.35 | 0.97 | 46000 | 91 |
| Example 108 | Par708 | 12 | 95 | 7 | 1.38 | 0.94 | 46000 | 93 |
| Example 109 | Par709 | 3 | 92 | 8 | 1.29 | 0.95 | 46000 | 92 |
| Example 110 | Par710 | 10 | 93 | 8 | 1.41 | 0.93 | 47000 | 92 |
| Example 111 | Par711 | 15 | 97 | 7 | 1.35 | 0.94 | 45000 | 89 |
| Example 112 | Par712 | 3 | 93 | 8 | 1.36 | 0.94 | 46000 | 93 |
| Example 113 | Par713 | 11 | 93 | 7 | 1.37 | 0.95 | 47000 | 92 |
| Example 114 | Par714 | 16 | 96 | 6 | 1.25 | 0.94 | 46000 | 91 |
| Example 115 | Par715 | 4 | 91 | 7 | 1.36 | 0.96 | 45000 | 89 |
| Example 116 | Par716 | 12 | 90 | 8 | 1.44 | 0.93 | 45000 | 92 |
| Example 117 | Par717 | 15 | 95 | 7 | 1.5 | 0.92 | 46000 | 93 |
| Example 118 | Par718 | 7 | 93 | 8 | 1.43 | 0.93 | 47000 | 92 |
| Example 119 | Par719 | 17 | 90 | 9 | 1.44 | 0.94 | 46000 | 91 |
| Example 120 | Par720 | 15 | 91 | 6 | 1.39 | 0.94 | 47000 | 93 |
| Example 121 | Par721 | 16 | 90 | 7 | 1.37 | 0.93 | 46000 | 89 |
| Example 122 | Par722 | 15 | 91 | 8 | 1.37 | 0.92 | 45000 | 90 |
| Example 123 | Par723 | 3 | 92 | 7 | 1.41 | 0.91 | 46000 | 91 |
| Example 124 | Par724 | 2 | 91 | 8 | 1.41 | 0.92 | 47000 | 91 |

Table 2-7 Evaluation Results

[Table 2-8]

| Table 2-8 Evaluation Results | | | | | | | | | | |
|---|---|---|---|---|---|---|---|---|---|---|
| | | Smoothness | | | Moist sensation | | | Softness | | |
| Class | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 101 | Par701 | 9 | 8 | 1 | 9 | 8 | 1 | 9 | 8 | 1 |
| Example 102 | Par702 | 9 | 8 | 1 | 9 | 7 | 2 | 9 | 7 | 2 |
| Example 103 | Par703 | 8 | 5 | 3 | 9 | 7 | 2 | 9 | 6 | 3 |
| Example 104 | Par704 | 10 | 10 | 0 | 10 | 10 | 0 | 10 | 10 | 0 |
| Example 105 | Par705 | 10 | 10 | 0 | 10 | 10 | 0 | 10 | 10 | 0 |
| Example 106 | Par706 | 10 | 10 | 0 | 10 | 10 | 0 | 10 | 10 | 0 |
| Example 107 | Par707 | 10 | 10 | 0 | 10 | 10 | 0 | 10 | 10 | 0 |
| Example 108 | Par708 | 10 | 10 | 0 | 10 | 10 | 0 | 10 | 10 | 0 |
| Example 109 | Par709 | 10 | 10 | 0 | 10 | 10 | 0 | 10 | 10 | 0 |
| Example 110 | Par710 | 10 | 10 | 0 | 10 | 10 | 0 | 10 | 10 | 0 |
| Example 111 | Par711 | 10 | 10 | 0 | 10 | 10 | 0 | 10 | 10 | 0 |
| Example 112 | Par712 | 10 | 10 | 0 | 10 | 10 | 0 | 10 | 10 | 0 |
| Example 113 | Par713 | 10 | 10 | 0 | 10 | 10 | 0 | 10 | 10 | 0 |
| Example 114 | Par714 | 10 | 10 | 0 | 10 | 10 | 0 | 10 | 10 | 0 |
| Example 115 | Par715 | 10 | 10 | 0 | 10 | 10 | 0 | 10 | 10 | 0 |
| Example 116 | Par716 | 9 | 8 | 1 | 8 | 8 | 0 | 9 | 8 | 1 |
| Example 117 | Par717 | 9 | 8 | 1 | 8 | 8 | 0 | 9 | 8 | 1 |
| Example 118 | Par718 | 9 | 8 | 1 | 8 | 8 | 0 | 9 | 8 | 1 |
| Example 119 | Par719 | 9 | 8 | 1 | 8 | 8 | 0 | 9 | 8 | 1 |
| Example 120 | Par720 | 9 | 8 | 1 | 8 | 8 | 0 | 9 | 8 | 1 |

(continued)

| Table 2-8 Evaluation Results | | | | | | | | | | |
|---|---|---|---|---|---|---|---|---|---|---|
| | | Smoothness | | | Moist sensation | | | Softness | | |
| Class | Particle number | Acceptable if the initial grade is 6 or higher and if the change is 4 or smaller | | | Acceptable if the initial grade is 6 or higher and if the change is 4 or smaller | | | Acceptable if the initial grade is 6 or higher and if the change is 4 or smaller | | |
| | | Initial | 96 hours | Change | Initial | 96 hours | Change | Initial | 96 hours | Change |
| Example 121 | Par721 | 9 | 8 | 1 | 8 | 8 | 0 | 9 | 8 | 1 |
| Example 122 | Par722 | 9 | 8 | 1 | 8 | 8 | 0 | 9 | 8 | 1 |
| Example 123 | Par723 | 10 | 10 | 0 | 10 | 10 | 0 | 10 | 10 | 0 |
| Example 124 | Par724 | 10 | 10 | 0 | 10 | 10 | 0 | 10 | 10 | 0 |

These results indicate that the cellulosic particles of the examples are highly biodegradable and exhibit little change in texture over time compared with those of the comparative examples.

Evaluations of Cosmetics

Production of Cosmetics

A variety of cosmetics are produced using the cellulosic particles of Examples and Comparative Examples indicated in Table 4. The specific processes are as follows.

Liquid Foundation

Liquid foundation is obtained by a known method according to the formula presented in Table 3-1.

[Table 3-1]

| Table 3-1 Liquid Foundation | | | |
|---|---|---|---|
| Formula | Compound | Product name (manufacturer) | Parts by mass |
| Particles | Particles | The cellulosic particles specified in Table 4 | 10 |
| Other ingredients | Propylene glycol | Propylene Glycol JSQI (Dow Toray) | 5 |
| | Bentonite | OVWIL BR (Mizusawa Industrial Chemicals) | 1 |
| | Triethanolamine | Triethanolamine 99% (Dow Toray) | 1 |
| | Stearic acid | NAA172 (NOF) | 3 |
| | Stearyl alcohol | NAA45 (NOF) | 1 |
| | Liquid paraffin | MORESCO-VIOLESS (MORESCO) | 8 |
| | Isopropyl myristate | IPM-R (NOF) | 5 |
| | Petrolatum | NOMCORT W (Nisshin OilliO) | 2 |
| | Stearic acid monoglyceride | EXCEL 84 (Kao Chemicals) | 2 |
| | POE (20) stearyl ether | EMALEX 602 (Nihon Emulsion) | 1 |
| | Titanium oxide | MKR-1 (Sakai Chemical) | 8 |
| | Kaolin | BERACLAY 20061 AMAZONIAN WHITE CLAY (BERECA) | 5 |

(continued)

| Table 3-1 Liquid Foundation | | | |
|---|---|---|---|
| Formula | Compound | Product name (manufacturer) | Parts by mass |
| | Iron oxide | C33-128 Sun CROMA RED Iron Oxide (Sun Chemical) | 0.5 |
| | Preservative | OPTIPHEN HD (Ashland Japan) | 0.5 |
| | Fragrance | Bisabolol rac. (BASF Japan) | 0.3 |
| | Purified water | | 46.5 |
| Total | | | 100 |

Milky Lotion

A milky lotion is obtained by a known method according to the formula presented in Table 3-2.

[Table 3-2]

| Table 3-2 Milky Lotion | | | |
|---|---|---|---|
| Formula | Compound | Product name (manufacturer) | Parts by mass |
| Particles | Particles | As in the Example or Comparative Example | 2 |
| Other ingredients | Propylene glycol | Propylene Glycol JSQI (Dow Toray) | 5 |
| | Polyethylene glycol 1500 | PEG#1500 (NOF) | 3 |
| | Carboxy vinyl polymer | NTC-CARBOMER 380 (Nikko Chemicals) | 0.1 |
| | Triethanolamine | Triethanolamine 99% (Dow Toray) | 1 |
| | Stearic acid | NAA172 (NOF) | 2 |
| | Cetyl alcohol | NAA44 (NOF) | 1.5 |
| | Liquid paraffin | MORESCO-VIOLESS (MORESCO) | 10 |
| | Petrolatum | NOMCORT W (Nisshin OilliO) | 3 |
| | Glyceryl oleate | NIKKOL MGO (Nikko Chemicals) | 1 |
| | POE (20) sorbitan oleate | NIKKOL TO -0V (Nikko Chemicals) | 1 |
| | Preservative | OPTIPHEN HD (Ashland Japan) | 0.2 |
| | Fragrance | Bisabolol rac. (BASF Japan) | 0.1 |
| | Purified water | | 70.1 |
| Total | | | 100 |

Loose Powder

A loose powder is obtained by mixing the ingredients listed in Table 3-3 in a blender, milling the mixture in a mill, and sieving the particles through a 250-$\mu$m mesh sieve.

[Table 3-3]

| Table 3-3 Loose Powder | | | |
|---|---|---|---|
| Formula | Compound | Product name (manufacturer) | Parts by mass |
| Particles | Particles | The cellulosic particles specified in Table 4 | 10 |

(continued)

| Table 3-3 Loose Powder | | | |
|---|---|---|---|
| Formula | Compound | Product name (manufacturer) | Parts by mass |
| Other ingredients | Talc | Talc CT-25 (Yamaguchi Mica) | 65 |
| | Kaolin | BERACLAY 20061 AMAZONIAN WHITE CLAY (BERECA) | 5 |
| | Titanium oxide | MKR-1 (Sakai Chemical) | 3 |
| | Zinc myristate | POWDER BASE M (NOF) | 5 |
| | Magnesium carbonate | Natrasorb HFB (Nouryon Japan) | 5 |
| | Sericite | Sericite FSE (Sanshin Mining Ind.) | 7 |
| Total | | | 100 |

Powder Foundation

Powder foundation is obtained by mixing the particles and powders according to the formula presented in Table 3-4, mixing binders according to the same, gradually adding the mixture of particles and powders into the binders with stirring, and then mixing the mixture.

[Table 3-4]

| Table 3-4 Powder Foundation | | | |
|---|---|---|---|
| Formula | Compound | Product name (manufacturer) | Parts by mass |
| Particles | Particles | The cellulosic particles specified in Table 4 | 8 |
| Other powders | Talc | Talc CT-25 (Yamaguchi Mica) | 52.5 |
| | Mica | Mica FA450 (Yamaguchi Mica) | 16 |
| | Titanium oxide | MKR-1 (Sakai Chemical) | 12 |
| | Black iron oxide | C33-134 Sun CROMA Black Iron Oxide (Sun Chemical) | 0.2 |
| | Red iron oxide | C33-128 Sun CROMA Red Iron Oxide (Sun Chemical) | 0.4 |
| | Yellow iron oxide | C33-210 Sun CROMA Yellow Iron Oxide (Sun Chemical) | 2.4 |
| Binders | Diisostearyl malate | Neosolue-DiSM (Nippon Fine Chemical) | 3 |
| | Caprylic/capric triglyceride | Caprylic/Capric Triglyceride (FUJIFILM Wako Pure Chemical) | 2 |
| | Neopentyl glycol dicaprate | NPDC (Kokyu Alcohol Kogyo) | 2 |
| | Pentylene glycol | DIOL PD (Kokyu Alcohol Kogyo) | 1.5 |
| Total | | | 100 |

Sunscreen Cream

According to the formula presented in Table 3-5, oil phase (1) is warmed to 50°C until dissolution, and the solution is mixed with oil phase (2). Water phase (2) is brought into dissolution, and the solution is mixed. The particles and the powders are added to the mixture of oil phases (1) and (2) and dispersed and mixed, and then the mixture of water phases (1) and (2) is added gradually for emulsification; this gives a sunscreen cream.

[Table 3-5]

| Table 3-5 Sunscreen Cream | | | |
|---|---|---|---|
| Formula | Compound | Product name (manufacturer) | Parts by mass |
| Particles | Particles | The cellulosic particles specified in Table 4 | 5 |

(continued)

| Table 3-5 Sunscreen Cream | | | |
|---|---|---|---|
| Formula | Compound | Product name (manufacturer) | Parts by mass |
| Other powders | Quaternium-18 hectorite | SUMECTON-SAN (Kunimine Industries) | 1 |
| | Titanium oxide | MKR-1 (Sakai Chemical) | 8 |
| Oil phase (1) | Ethylhexyl methoxycinnamate | Uvinul MC80 (BASF Japan) | 4 |
| | t-Butyl methoxydibenzoylmethane | Eusolex 9030 (Merck KGaA) | 0.5 |
| | Bis-ethylhexyloxyphenol methoxyphenyl triazine | Tinosorb S (BASF Japan) | 2 |
| | Isopropyl sebacate | Isopropyl Sebacate (FUJIFILM Wako Pure Chemical) | 6 |
| | Caprylic/capric triglyceride | Caprylic/Capric Triglyceride (FUJIFILM Wako Pure Chemical) | 2 |
| Oil phase (2) | Cetyl PEG/PPG-10/1 dimethicone | KF-6048 (Shin-Etsu Chemical) | 4 |
| | Sorbitan isostearate | EMALEX SPIS 100 (Nihon Emulsion) | 0.4 |
| | Cyclopentasiloxane | KF-995 (Shin-Etsu Chemical) | 16 |
| | Ethylhexylglycerin, glyceryl caprylate | NIKKOL NIKKOGUARD 88 (Nikko Chemicals) | 0.4 |
| Water phase (1) | PEG-240/HDI copolymer bis-decyltetradeceth-20 ether | ADEKA NOL GT 700 | 1 |
| | Glycerin | RG-CO-P (NOF) | 4 |
| | 1,3-Butylene glycol | HAISUGARCANE BG (Kokyu Alcohol Kogyo) | 4 |
| | Pentylene glycol | DIOL PD (Kokyu Alcohol Kogyo) | 1 |
| | Phenoxyethanol | Phenoxetol (Clariant Japan) | 0.3 |
| Water phase (2) | Magnesium sulfate | Magnesium Sulfate (FUJIFILM Wako Pure Chemical) | 0.3 |
| | Purified water | | 40.1 |
| Total | | | 100 |

All-in-One Gel

According to the formula presented in Table 3-6, water phases (1) and (2) are mixed together. Then oil phase (1) is mixed and added to the mixture of water phases (1) and (2). Oil phase (2) is warmed to 70°C, and the particles are added to it; this gives a dispersion. The resulting dispersion is added to the mixture of water phases (1) and (2) and oil phase (1), and the resulting mixture is stirred and mixed for emulsification. Stirring the emulsion with the neutralizing agent and cooling the mixture gives an all-in-one gel.

[Table 3-6]

| Table 3-6 All-in-One Gel | | | |
|---|---|---|---|
| Formula | Compound | Product name (manufacturer) | Parts by mass |
| Particles | Particles | The cellulosic particles specified in Table 4 | 4 |

(continued)

| Table 3-6 All-in-One Gel | | | |
|---|---|---|---|
| Formula | Compound | Product name (manufacturer) | Parts by mass |
| Oil phase (1) | Xanthan gum | NOMCORT Z (The Nisshin OilliO Group) | 0.1 |
| | Hydrogenated lecithin | COATSOME NC-21 (NOF) | 0.1 |
| | Glycerin | RG-CO-P (NOF) | 5 |
| | Isopentyldiol | Isoprene Glycol (Kuraray) | 4 |
| Oil phase (2) | Polyglyceryl-10 isostearate | Sunsoft Q-18S-C (Taiyo Kagaku) | 1.2 |
| | Polyglyceryl-4 isostearate | NIKKOL Tetraglyn 1-SV (Nikko Chemicals) | 0.3 |
| | Behenyl alcohol | NAA-422 (NOF) | 1.8 |
| | Octyldodecanol | RISONOL 20SP (Kokyu Alcohol Kogyo) | 0.8 |
| | Cetyl ethylhexanoate | FineNeo-CIO (Nippon Fine Chemical) | 3.2 |
| | Squalane | NIKKOL Olive Squalane (Nikko Chemicals) | 0.6 |
| | Tocopherol | Tocopherol 100 (The Nisshin OilliO Group) | 0.6 |
| | Ethylhexylglycerin, glyceryl caprylate | NIKKOL NIKKOGUARD 88 (Nikko Chemicals) | 0.6 |
| Water phase (1) | Carboxy vinyl polymer | NTC-CARBOMER 380 (Nikko Chemicals) | 0.4 |
| | Pentylene glycol | DIOL PD (Kokyu Alcohol Kogyo) | 1 |
| | Phenoxyethanol | Phenoxetol (Clariant Japan) | 0.3 |
| | Sodium dilauramidoglutamide lysine, water | Pellicer LB 100 (Asahi Kasei Finechem) | 0.1 |
| Water phase (2) | Citric acid | Citric Acid (FUJIFILM Wako Pure Chemical) | 0.1 |
| | Purified water | | 1.4 |
| Neutralizing agent | A 10% aqueous solution of sodium hydroxide | | |
| Total | | | 100 |

Foundation Primer

According to the formula presented in Table 3-7, the particles are dispersed in component A, and the resulting mixture is stirred. Adding component B and stirring the resulting mixture gives a foundation primer.

[Table 3-7]

| Table 3-7 Foundation Primer | | | |
|---|---|---|---|
| Formula | Compound | Product name (manufacturer) | Parts by mass |
| Particles | Particles | The cellulosic particles specified in Table 4 | 10 |

(continued)

| Table 3-7 Foundation Primer | | | |
|---|---|---|---|
| Formula | Compound | Product name (manufacturer) | Parts by mass |
| Component A | Dimethicone/PEG-10/15 crosspolymer, dimethicone | KSG-210 (Shin-Etsu Chemical) | 3.5 |
| | PEG-9 polydimethylsiloxyethyl dimethicone | KF-6028 (Shin-Etsu Chemical) | 2 |
| | Dimethicone | KF-7312K (Shin-Etsu Chemical) | 5 |
| | Isononyl isononanoate | KAK99 (Kokyu Alcohol Kogyo) | 4.5 |
| | Ethylhexyl methoxycinnamate | NOMCORT TAB (The Nisshin OilliO Group) | 10 |
| | Quaternium-18 hectorite | SUMECTON-SAN (Kunimine Industries) | 1.2 |
| | Dimethicone/vinyl dimethicone crosspolymer, dimethicone | KSG-16 (Shin-Etsu Chemical) | 5 |
| | Cyclomethicone | DOWSIL SH245 Fluid (Dow Toray) | 25 |
| Component B | 1,3-Butylene glycol | HAISUGARCANE BG (Kokyu Alcohol Kogyo) | 5 |
| | Sodium citrate | Trisodium Citrate (Jungbunzlauer International AG) | 2 |
| | Preservative | OPTIPHEN HD (Ashland Japan) | 0.3 |
| | Purified water | | 26.5 |
| Total | | | 100 |

Lip Primer

According to the formula presented in Table 3-8, component B is heated to 60°C and mixed. The particles are dispersed in the mixture, the resulting dispersion is microwaved with component A until dissolution, and the solution is mixed and then cooled in a mold. Enclosing the resulting solid into a lipstick case gives a lip primer.

[Table 3-8]

| Table 3-8 Lip Primer | | | |
|---|---|---|---|
| Formula | Compound | Product name (manufacturer) | Parts by mass |
| Particles | Particles | The cellulosic particles specified in Table 4 | 10 |
| Component A | Ceresin | CERESIN #810 (Nikko Rika) | 4.27 |
| | Microcrystalline wax | Refined Microcrystalline Wax (Nikko Rika) | 1.55 |
| | Candelilla wax | Refined Candelilla Wax No. 1 (Nippon Wax) | 5.03 |
| | Paraffin | Refined Paraffin Wax (Nikko Rika) | 3.07 |

(continued)

| Table 3-8 Lip Primer | | | |
|---|---|---|---|
| Formula | Compound | Product name (manufacturer) | Parts by mass |
| Component B | Diisostearyl malate | Neosolue-DiSM (Nippon Fine Chemical) | 17.95 |
| | Dipentaerythrite fatty acid ester | COSMOL 168 EV (The Nisshin OilliO Group) | 6.22 |
| | Adsorption refined lanolin | SUPER STEROL LIQUID (Croda Japan) | 2.52 |
| | Liquid lanolin acetate | ACELAN SP (Croda Japan) | 13.34 |
| | Ethylhexylglyceryl | GLYMOIST (NOF) | 19.02 |
| | Liquid paraffin | HYDROBRITE 380 PO (Sonneborn) | 7.28 |
| | Isotridecyl isononanoate | KAK139 (Kokyu Alcohol Kogyo) | 3.21 |
| | Polyglyceryl-2 triisostearate | EMALEX TISG-2 (Nihon Emulsion) | 4.01 |
| | Methylphenyl polysiloxane | BELSIL PDM 20 (Wacker Asahikasei Silicone) | 2.41 |
| | Methylparaben | Nipagin M (Clariant Japan) | 0.07 |
| | Tocopherol | Tocopherol 100 (The Nisshin OilliO Group) | 0.05 |
| Total | | | 100 |

Body Powder

A body powder is obtained by mixing the ingredients listed in Table 3-9 together.

[Table 3-9]

| Table 3-9 Body Powder | | | |
|---|---|---|---|
| Formula | Compound | Product name (manufacturer) | Parts by mass |
| Particles | Particles | The cellulosic particles specified in Table 4 | 10 |
| Other ingredients | Talc | Talc CT-25 (Yamaguchi Mica) | 89.7 |
| | Fragrance | Bisabolol rac. (BASF Japan) | 0.3 |

Solid Powder Eyeshadow

According to the formula presented in Table 3-10, the particles and powders are mixed together, and the mixed powder is further mixed with a homogeneous solution of the binder; shaping the mixture by compression molding gives a solid powder eyeshadow.

[Table 3-10]

| Table 3-10 Solid Powder Eyeshadow | | | |
|---|---|---|---|
| Formula | Compound | Product name (manufacturer) | Parts by mass |
| Particles | Particles | The cellulosic particles specified in Table 4 | 51 |
| Other powders | Mica | Talc CT-25 (Yamaguchi Mica) | 15 |
| | Sericite | Sericite FSE (Sanshin Mining Ind.) | 5 |
| | Pigment | Unipure Blue LC 621 (Sensient Technologies Japan) | 15 |
| | Pearl pigment | TWINCLEPEARL (Nihon Koken Kogyo | 10 |
| Binder | Methyl polysiloxane | BELSIL DM 10 (Wacker Asahikasei Silicone) | 2 |
| Others | Sorbitan sesquioleate | EMALEX SPO-150 (Nihon Emulsion) | 2 |
| Total | | | 100 |

Evaluations

The resulting cosmetics are subjected to the above-described texture evaluations (smoothness, moist sensation, and softness) after 24 hours of storage in a temperature-controlled chamber at a low temperature (0°C) and after 24 hours of storage in a temperature-controlled chamber at a high temperature (60°C).

[Table 4-1]

Table 4-1

| Class | Particle number | Liquid foundation | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 7 |
| Example 2 | Par02 | 6 | 7 | 6 | 6 | 7 | 7 |
| Example 3 | Par03 | 6 | 7 | 6 | 6 | 7 | 7 |
| Example 4 | Par04 | 6 | 7 | 6 | 6 | 7 | 7 |
| Example 5 | Par05 | 5 | 7 | 5 | 6 | 7 | 6 |
| Example 26 | Par026 | 8 | 7 | 7 | 8 | 8 | 7 |
| Example 41 | Par041 | 6 | 7 | 6 | 6 | 7 | 7 |
| Example 54 | Par054 | 8 | 7 | 7 | 8 | 8 | 7 |
| Example 55 | Par055 | 6 | 7 | 6 | 6 | 7 | 7 |
| Example 56 | Par056 | 8 | 7 | 7 | 8 | 8 | 7 |
| Example 57 | Par057 | 6 | 7 | 6 | 6 | 7 | 7 |
| Example 58 | Par058 | 8 | 7 | 7 | 8 | 8 | 7 |
| Example 59 | Par059 | 6 | 7 | 6 | 6 | 7 | 7 |
| Example 60 | Par060 | 8 | 7 | 7 | 8 | 8 | 7 |
| Example 61 | Par061 | 6 | 7 | 6 | 6 | 7 | 7 |
| Example 70 | Par70 | 8 | 8 | 8 | 9 | 9 | 8 |
| Example 75 | Par75 | 8 | 7 | 7 | 8 | 8 | 7 |
| Example 77 | Par77 | 8 | 8 | 8 | 9 | 9 | 8 |
| Example 101 | Par701 | 8 | 8 | 8 | 9 | 9 | 8 |
| Example 102 | Par702 | 8 | 7 | 7 | 8 | 8 | 7 |
| Example 103 | Par703 | 5 | 7 | 5 | 6 | 7 | 6 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |

(continued)

| Class | Particle number | Liquid foundation | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 9 | 9 | 8 |
| Example 117 | Par717 | 8 | 8 | 8 | 9 | 9 | 8 |
| Example 118 | Par718 | 8 | 8 | 8 | 9 | 9 | 8 |
| Example 119 | Par719 | 8 | 8 | 8 | 9 | 9 | 8 |
| Example 120 | Par720 | 8 | 8 | 8 | 9 | 9 | 8 |
| Example 121 | Par721 | 8 | 8 | 8 | 9 | 9 | 8 |
| Example 122 | Par722 | 8 | 8 | 8 | 9 | 9 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par 10 1 | 3 | 7 | 3 | 4 | 6 | 3 |
| Comparative Example 2 | Par102 | 5 | 4 | 4 | 5 | 5 | 5 |
| Comparative Example 3 | Par103 | 5 | 5 | 5 | 4 | 5 | 5 |
| Comparative Example 4 | Par104 | 4 | 7 | 3 | 4 | 6 | 3 |
| Comparative Example 9 | Par115 | 5 | 3 | 5 | 5 | 4 | 5 |
| Comparative Example 10 | Par 116 | 5 | 5 | 5 | 5 | 4 | 5 |
| Comparative Example 11 | Par 117 | 5 | 5 | 5 | 5 | 4 | 5 |

[Table 4-2]

Table 4-2

| Class | Particle number | Milky lotion | | | | | |
|---|---|---|---|---|---|---|---|
| | | Low temperature, 0°C | | | High temperature, 60°C | | |
| | | Smoothness | |Moist sensation | Softness | Smoothness | Moist sensation | Softness |
| Example 1 | Par01 | 6 | 7 | 6 | 6 | 7 | 7 |
| Example 2 | Par02 | 6 | 7 | 6 | 6 | 7 | 7 |
| Example 3 | Par03 | 6 | 7 | 6 | 6 | 7 | 7 |

(continued)

| Class | Particle number | Milky lotion | | | | | |
|---|---|---|---|---|---|---|---|
| | | Low temperature, 0°C | | | High temperature, 60°C | | |
| | | Smoothness | \|Moist sensation | Softness | Smoothness | Moist sensation | Softness |
| Example 4 | Par04 | 6 | 7 | 6 | 6 | 7 | 7 |
| Example 5 | Par05 | 5 | 7 | 5 | 6 | 7 | 6 |
| Example 26 | Par026 | 8 | 7 | 7 | 8 | 8 | 7 |
| Example 41 | Par041 | 6 | 7 | 6 | 6 | 7 | 7 |
| Example 54 | Par054 | 8 | 7 | 7 | 8 | 8 | 7 |
| Example 55 | Par055 | 6 | 7 | 6 | 6 | 7 | 7 |
| Example 56 | Par056 | 8 | 7 | 7 | 8 | 8 | 7 |
| Example 57 | Par057 | 6 | 7 | 6 | 6 | 7 | 7 |
| Example 58 | Par058 | 8 | 7 | 7 | 8 | 8 | 7 |
| Example 59 | Par059 | 6 | 7 | 6 | 6 | 7 | 7 |
| Example 60 | Par060 | 8 | 7 | 7 | 8 | 8 | 7 |
| Example 61 | Par061 | 6 | 7 | 6 | 6 | 7 | 7 |
| Example 70 | Par70 | 8 | 8 | 8 | 9 | 9 | 8 |
| Example 75 | Par75 | 8 | 7 | 7 | 8 | 8 | 7 |
| Example 77 | Par77 | 8 | 8 | 8 | 9 | 9 | 8 |
| Example 101 | Par701 | 8 | 8 | 8 | 9 | 9 | 8 |
| Example 102 | Par702 | 8 | 7 | 7 | 8 | 8 | 7 |
| Example 103 | Par703 | 5 | 7 | 5 | 6 | 7 | 6 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 101 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 9 | 9 | 8 |
| Example 117 | Par717 | 8 | 8 | 8 | 9 | 9 | 8 |
| Example 118 | Par718 | 8 | 8 | 8 | 9 | 9 | 8 |
| Example 119 | Par719 | 8 | 8 | 8 | 9 | 9 | 8 |
| Example 120 | Par720 | 8 | 8 | 8 | 9 | 9 | 8 |
| Example 121 | Par721 | 8 | 8 | 8 | 9 | 9 | 8 |
| Example 122 | Par722 | 8 | 8 | 8 | 9 | 9 | 8 |

(continued)

| Class | Particle number | Milky lotion | | | | | |
|---|---|---|---|---|---|---|---|
| | | Low temperature, 0°C | | | High temperature, 60°C | | |
| | | Smoothness | \|Moist sensation | Softness | Smoothness | Moist sensation | Softness |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 31 | 7 | 3 | 4 | 6 | 3 |
| Comparative Example 2 | Par102 | 5 | 4 | 4 | 5 | 5 | 5 |
| Comparative Example 3 | Par103 | 5 | 5 | 5 | 4 | 5 | 5 |
| Comparative Example 4 | Par104 | 4 | 7 | 3 | 4 | 6 | 3 |
| Comparative Example 9 | Par115 | 5 | 3 | 5 | 5 | 4 | 5 |
| Comparative Example 10 | Par116 | 5 | 5 | 5 | 5 | 4 | 5 |
| Comparative Example 11 | Par117 | 5 | 5 | 5 | 5 | 4 | 5 |

[Table 4-3]

Table 4-3

| Class | Particle number | Loose powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Par054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |

(continued)

| Class | Particle number | Loose powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |
| Comparative Example 4 | Par104 | 3 | 7 | 3 | 3 | 6 | 3 |
| Comparative Example 9 | Par115 | 5 | 4 | 5 | 5 | 3 | 4 |

(continued)

| Class | Particle number | Loose powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Comparative Example 10 | Par116 | 5 | 4 | 4 | 4 | 4 | 4 |
| Comparative Example 11 | Par117 | 5 | 4 | 4 | 4 | 4 | 4 |

[Table 4-4]

Table 4-4

| Class | Particle number | Powder foundation | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Pai054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |

(continued)

| Class | Particle number | Powder foundation | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |
| Comparative Example 4 | Par104 | 3 | 7 | 3 | 3 | 6 | 3 |
| Comparative Example 9 | Par115 | 5 | 4 | 5 | 5 | 3 | 4 |
| Comparative Example 10 | Par116 | 5 | 4 | 4 | 4 | 4 | 4 |
| Comparative Example 11 | Par117 | 5 | 4 | 4 | 4 | 4 | 4 |

[Table 4-5]

Table 4-5

| Class | Particle number | Sunscreen cream | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 7 |
| Example 2 | Par02 | 6 | 7 | 6 | 6 | 7 | 7 |
| Example 3 | Par03 | 6 | 7 | 6 | 6 | 7 | 7 |
| Example 4 | Par04 | 6 | 7 | 6 | 6 | 7 | 7 |
| Example 5 | Par05 | 5 | 7 | 5 | 6 | 7 | 6 |
| Example 26 | Par026 | 8 | 7 | 7 | 8 | 8 | 7 |
| Example 41 | Par041 | 6 | 7 | 6 | 6 | 7 | 7 |
| Example 54 | Par054 | 8 | 7 | 7 | 8 | 8 | 7 |
| Example 55 | Par055 | 6 | 7 | 6 | 6 | 7 | 7 |
| Example 56 | Par056 | 8 | 7 | 7 | 8 | 8 | 7 |
| Example 57 | Par057 | 6 | 7 | 6 | 6 | 7 | 7 |
| Example 58 | Par058 | 8 | 7 | 7 | 8 | 8 | 7 |
| Example 59 | Par059 | 6 | 7 | 6 | 6 | 7 | 7 |
| Example 60 | Par060 | 8 | 7 | 7 | 8 | 8 | 7 |
| Example 61 | Par061 | 6 | 7 | 6 | 6 | 7 | 7 |
| Example 70 | Par70 | 9 | 8 | 8 | 9 | 9 | 8 |
| Example 75 | Par75 | 8 | 7 | 7 | 8 | 8 | 7 |
| Example 77 | Par77 | 9 | 8 | 8 | 9 | 9 | 8 |
| Example 101 | Par701 | 9 | 8 | 8 | 9 | 9 | 8 |
| Example 102 | Par702 | 8 | 7 | 7 | 8 | 8 | 7 |
| Example 103 | Par703 | 5 | 7 | 5 | 6 | 7 | 6 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 9 | 8 | 8 | 9 | 9 | 8 |
| Example 117 | Par717 | 9 | 8 | 8 | 9 | 9 | 8 |
| Example 118 | Par718 | 9 | 8 | 8 | 9 | 9 | 8 |
| Example 119 | Par719 | 9 | 8 | 8 | 9 | 9 | 8 |

(continued)

| Class | Particle number | Sunscreen cream | | | | | |
| --- | --- | --- | --- | --- | --- | --- | --- |
| | | Low temperature, 0°C | | | High temperature, 60°C | | |
| | | Smoothness | Moist sensation | Softness | Smoothness | Moist sensation | Softness |
| Example 120 | Par720 | 9 | 8 | 8 | 9 | 9 | 8 |
| Example 121 | Par721 | 9 | 8 | 8 | 9 | 9 | 8 |
| Example 122 | Par722 | 9 | 8 | 8 | 9 | 9 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 3 | 7 | 3 | 4 | 6 | 3 |
| Comparative Example 2 | Par102 | 5 | 4 | 4 | 5 | 5 | 5 |
| Comparative Example 3 | Par103 | 5 | 5 | 5 | 4 | 5 | 5 |
| Comparative Example 4 | Par104 | 4 | 7 | 3 | 4 | 6 | 3 |
| Comparative Example 9 | Par115 | 5 | 3 | 5 | 5 | 4 | 5 |
| Comparative Example 10 | Par116 | 5 | 5 | 5 | 5 | 4 | 5 |
| Comparative Example 11 | Par117 | 5 | 5 | 5 | 5 | 4 | 5 |

[Table 4-6]

Table 4-6

| Class | Particle number | All-in-one gel | | | | | |
| --- | --- | --- | --- | --- | --- | --- | --- |
| | | Low temperature, 0°C | | | High temperature, 60°C | | |
| | | Smoothness | [Moist sensation | Softness | Smoothness | Moist sensation | Softness |
| Example 1 | Par01 | 6 | 7 | 6 | 6 | 7 | 7 |
| Example 2 | Par02 | 6 | 7 | 6 | 6 | 7 | 7 |
| Example 3 | Par03 | 6 | 7 | 6 | 6 | 7 | 7 |
| Example 4 | Par04 | 6 | 7 | 6 | 6 | 7 | 7 |
| Example 5 | Par05 | 5 | 7 | 5 | 6 | 7 | 6 |
| Example 26 | Par026 | 8 | 7 | 7 | 8 | 8 | 7 |
| Example 41 | Par041 | 6 | 7 | 6 | 6 | 7 | 7 |
| Example 54 | Par054 | 8 | 7 | 7 | 8 | 8 | 7 |
| Example 55 | Par055 | 6 | 7 | 6 | 6 | 7 | 7 |
| Example 56 | Par056 | 8 | 7 | 7 | 8 | 8 | 7 |
| Example 57 | Par057 | 6 | 7 | 6 | 6 | 7 | 7 |

(continued)

| Class | Particle number | All-in-one gel | | | | | |
|---|---|---|---|---|---|---|---|
| | | Low temperature, 0°C | | | High temperature, 60°C | | |
| | | Smoothness | [Moist sensation | Softness | Smoothness | Moist sensation | Softness |
| Example 58 | Par058 | 8 | 7 | 7 | 8 | 8 | 7 |
| Example 59 | Par059 | 6| | 7 | 6 | 6 | 7 | 7 |
| Example 60 | Par060 | 8 | 7 | 7 | 8 | 8 | 7 |
| Example 61 | Par061 | 6 | 7 | 6 | 6 | 7 | 7 |
| Example 70 | Par70 | 8 | 8 | 8 | 9 | 9 | 8 |
| Example 75 | Par75 | 8 | 7 | 7 | 8 | 8 | 7 |
| Example 77 | Par77 | 8 | 8 | 8 | 9 | 9 | 8 |
| Example 101 | Par701 | 8 | 8 | 8 | 9 | 9 | 8 |
| Example 102 | Par702 | 8 | 7 | 7 | 8 | 8 | 7 |
| Example 103 | Par703 | 5 | 7 | 5 | 6 | 7 | 6 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 9 | 9 | 8 |
| Example 117 | Par717 | 8 | 8 | 8 | 9 | 9 | 8 |
| Example 118 | Par718 | 8 | 8 | 8 | 9 | 9 | 8 |
| Example 119 | Par719 | 8 | 8 | 8 | 9 | 9 | 8 |
| Example 120 | Par720 | 8 | 8 | 8 | 9 | 9 | 8 |
| Example 121 | Par721 | 8 | 8 | 8 | 9 | 9 | 8 |
| Example 122 | Par722 | 8 | 8 | 8 | 9 | 9 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 3 | 7 | 3 | 4 | 6 | 3 |
| Comparative Example 2 | Par102 | 5 | 4 | 4 | 5 | 5 | 5 |
| Comparative Example 3 | Par103 | 5 | 5 | 5 | 4 | 5 | 5 |

(continued)

| Class | Particle number | All-in-one gel | | | | | |
|---|---|---|---|---|---|---|---|
| | | Low temperature, 0°C | | | High temperature, 60°C | | |
| | | Smoothness | [Moist sensation | Softness | Smoothness | Moist sensation | Softness |
| Comparative Example 4 | Par104 | 4 | 7 | 3 | 4 | 6 | 3 |
| Comparative Example 9 | Par115 | 5 | 3 | 5 | 5 | 4 | 5 |
| Comparative Example 10 | Par116 | 5 | 5 | 5 | 5 | 4 | 5 |
| Comparative Example 11 | Par117 | 5 | 5 | 5 | 5 | 4 | 5 |

[Table 4-7]

Table 4-7

| Class | Particle number | Foundation primer | | | | | |
|---|---|---|---|---|---|---|---|
| | | Low temperature, 0°C | | | High temperature, 60°C | | |
| | | Smoothness Moist | sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Par054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |

(continued)

| Class | Particle number | Foundation primer | | | | | |
|-------|-----------------|-------------------|---|---|---|---|---|
| | | Low temperature, 0°C | | | High temperature, 60°C | | |
| | | Smoothness Moist | sensation | Softness | Smoothness | Moist sensation | Softness |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 101 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |
| Comparative Example 4 | Par104 | 3 | 7 | 3 | 3 | 6 | 3 |
| Comparative Example 9 | Par115 | 5 | 4 | 5 | 5 | 3 | 4 |
| Comparative Example 10 | Par116 | 5 | 4 | 4 | 4 | 4 | 4 |
| Comparative Example 11 | Par117 | 5 | 4 | 4 | 4 | 4 | 4 |

[Table 4-8]

Table 4-8

| Class | Particle number | Lip primer | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Par054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |

(continued)

| Class | Particle number | Lip primer | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |
| Comparative Example 4 | Par104 | 3 | 7 | 3 | 3 | 6 | 3 |
| Comparative Example 9 | Par115 | 5 | 4 | 5 | 5 | 3 | 4 |
| Comparative Example 10 | Par116 | 5 | 4 | 4 | 4 | 4 | 4 |
| Comparative Example 11 | Par117 | 5 | 4 | 4 | 4 | 4 | 4 |

[Table 4-9]

Table 4-9

| Class | Particle number | Body powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Par054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |

(continued)

| Class | Particle number | Body powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |

(continued)

| Class | Particle number | Body powder | | | | | |
|---|---|---|---|---|---|---|---|
| | | Low temperature, 0°C | | | High temperature, 60°C | | |
| | | Smoothness | Moist sensation | Softness | Smoothness | Moist sensation | Softness |
| Comparative Example 4 | Par104 | 3 | 7 | 3 | 3 | 6 | 3 |
| Comparative Example 9 | Par115 | 5 | 4 | 5 | 5 | 3 | 4 |
| Comparative Example 10 | Par116 | 5 | 4 | 4 | 4 | 4 | 4 |
| Comparative Example 11 | Par117 | 5 | 4 | 4 | 4 | 4 | 4 |

[Table 4-10]

Table 4-10

| Class | Particle number | Solid powder eyeshadow | | | | | |
|---|---|---|---|---|---|---|---|
| | | Low temperature, 0°C | | | High temperature, 60°C | | |
| | | Smoothness | \|Moist sensation | Softness | Smoothness | Moist sensation | Softness |
| Example 1 | Par01 | 6 | 7 | 6 | 6 | 7 | 6 |
| Example 2 | Par02 | 6 | 7 | 6 | 6 | 7 | 6 |
| Example 3 | Par03 | 6 | 7 | 6 | 6 | 7 | 6 |
| Example 4 | Par04 | 6 | 7 | 6 | 6 | 7 | 6 |
| Example 5 | Par05 | 5 | 7 | 5 | 6 | 7 | 5 |
| Example 26 | Par026 | 7 | 7 | 7 | 7 | 8 | 7 |
| Example 41 | Par041 | 6 | 7 | 6 | 6 | 7 | 6 |
| Example 54 | Par054 | 7 | 7 | 7 | 7 | 8 | 7 |
| Example 55 | Par055 | 6 | 7 | 6 | 6 | 7 | 6 |
| Example 56 | Par056 | 7 | 7 | 7 | 7 | 8 | 7 |
| Example 57 | Par057 | 6 | 7 | 6 | 6 | 7 | 6 |
| Example 58 | Par058 | 7 | 7 | 7 | 7 | 8 | 7 |
| Example 59 | Par059 | 6 | 7 | 6 | 6 | 7 | 6 |
| Example 60 | Par060 | 7 | 7 | 7 | 7 | 8 | 7 |
| Example 61 | Par061 | 6 | 7 | 6 | 6 | 7 | 6 |
| Example 70 | Par70 | 8 | 8 | 8 | 8 | 8 | 8 |
| Example 75 | Par75 | 7 | 7 | 7 | 7 | 8 | 7 |
| Example 77 | Par77 | 8 | 8 | 8 | 8 | 8 | 8 |
| Example 101 | Par701 | 8 | 8 | 8 | 8 | 8 | 8 |
| Example 102 | Par702 | 7 | 7 | 7 | 7 | 8 | 7 |
| Example 103 | Par703 | 5 | 7 | 5 | 6 | 7 | 5 |

(continued)

| Class | Particle number | Solid powder eyeshadow | | | | | |
|---|---|---|---|---|---|---|---|
| | | Low temperature, 0°C | | | High temperature, 60°C | | |
| | | Smoothness | \|Moist sensation | Softness | Smoothness | Moist sensation | Softness |
| Example 104 | Par704 | 10 | 10 | 10 | 10 | 10 | 10 |
| Example 105 | Par705 | 10 | 10 | 10 | 10 | 10 | 10 |
| Example 106 | Par706 | 10 | 10 | 10 | 10 | 10 | 10 |
| Example 107 | Par707 | 10 | 10 | 10 | 10 | 10 | 10 |
| Example 108 | Par708 | 10 | 10 | 10 | 10 | 10 | 10 |
| Example 109 | Par709 | 10 | 10 | 10 | 10 | 10 | 10 |
| Example 110 | Par710 | 10 | 10 | 10 | 10 | 10 | 10 |
| Example 111 | Par711 | 10 | 10 | 10 | 10 | 10 | 10 |
| Example 112 | Par712 | 10 | 10 | 10 | 10 | 10 | 10 |
| Example 113 | Par713 | 10 | 10 | 10 | 10 | 10 | 10 |
| Example 114 | Par714 | 10 | 10 | 10 | 10 | 10 | 10 |
| Example 115 | Par715 | 10 | 10 | 10 | 10 | 10 | 10 |
| Example 116 | Par716 | 8 | 8 | 8 | 8 | 8 | 8 |
| Example 117 | Par717 | 8 | 8 | 8 | 8 | 8 | 8 |
| Example 118 | Par718 | 8 | 8 | 8 | 8 | 8 | 8 |
| Example 119 | Par719 | 8 | 8 | 8 | 8 | 8 | 8 |
| Example 120 | Par720 | 8 | 8 | 8 | 8 | 8 | 8 |
| Example 121 | Par721 | 8 | 8 | 8 | 8 | 8 | 8 |
| Example 122 | Par722 | 8 | 8 | 8 | 8 | 8 | 8 |
| Example 123 | Par723 | 10 | 10 | 10 | 10 | 10 | 10 |
| Example 124 | Par724 | 10 | 10 | 10 | 10 | 10 | 10 |
| Comparative Example 1 | Par101 | 2 | 6 | 3 | 3 | 6 | 3 |
| Comparative Example 2 | Par102 | 5 | 6 | 5 | 5 | 5 | 5 |
| Comparative Example 3 | Par103 | 5 | 5 | 5 | 5 | 5 | 5 |
| Comparative Example 4 | Par104 | 3 | 7 | 3 | 3 | 6 | 3 |
| Comparative Example 9 | Par115 | 5 | 4 | 5 | 5 | 3 | 4 |
| Comparative Example 10 | Par116 | 5 | 4 | 4 | 4 | 4 | 4 |
| Comparative Example 11 | Par117 | 5 | 4 | 4 | 4 | 4 | 4 |

These results indicate that cosmetics made with cellulosic particles of examples, compared with those of comparative examples, produce superior skin feelings (smoothness, moist sensation, and softness) even at high or low temperatures.

**Claims**

1. A cellulosic particle comprising:

    a core particle containing cellulose as a base constituent; and
    a coating layer covering the core particle, having:

        a first coating layer covering the core particle, and
        a second coating layer covering the first coating layer,
        wherein 5-day and 60-day percentage biodegradations of the cellulosic particle measured as per JIS K6950:2000 are lower than 20% and 60% or higher, respectively,
        wherein the core particle is produced from cellulose particle precursors that are prepared from an emulsion of a cellulose acylate solution in ethyl acetate and a carboxymethyl cellulose dispersion in methyl ethyl ketone that has been heated to 80°C with sodium hydroxide and then treated with hydrochloric acid and then saponified,
        wherein the first coating layer and the second coating layer contain

        a polyamine compound and a wax, respectively, or
        a linear-chain fatty acid and a wax, respectively, or
        a polyamine compound and a linear-chain fatty acid, respectively, or
        a polyamine compound and an amino acid compound, respectively, or
        an arginine compound and a linear-chain fatty acid metallic salt, respectively, or
        an arginine compound and a linear-chain fatty acid, respectively, or
        an arginine compound and an amino acid compound, respectively, or
        an arginine compound and a wax, respectively, or
        a polyamine compound and a linear-chain fatty acid metallic salt, respectively,
        wherein the polyamine compound is a polyalkyleneimine, polyallylamine, polyvinylamine, or polylysine,
        the linear-chain fatty acid is behenic acid, arachidic acid, or palmitic acid,
        the arginine compound is L-arginine, D-arginine, 2-amino-3-methyl-5-guanidinopentanoic acid, 2-amino-3-ethyl-5-guanidinopentanoic acid, or 2-amino-3,3-dimethyl-5-guanidinopentanoic acid,
        the wax is castor oil, paulownia oil, linseed oil, shortening, corn oil, soybean oil, sesame oil, rapeseed oil, sunflower oil, rice bran oil, camellia oil, coconut oil, palm oil, walnut oil, olive oil, peanut oil, almond oil, jojoba oil, cocoa butter, shea butter, neem oil, safflower oil, Japan wax, candelilla wax, rice bran wax, carnauba wax, Rosa damascena flower wax, petroleum waxes, synthetic hydrocarbon waxes, malic acid, glutaric acid, adipic acid, azelaic acid, sebacic acid, dodecanedioic acid, or C10 to C25 alcohols,
        the amino acid compound is lauryl leucine, lauryl arginine, or myristyl leucine,
        the linear-chain fatty acid metallic salt is the metallic salts of C10 to C25 fatty acids which is metallic salts of stearic acid, palmitic acid, lauric acid, oleic acid, linoleic acid, and ricinoleic acid, and metals in linear-chain fatty acid metallic salts includes magnesium, calcium, aluminum, barium, or zinc.

2. The cellulosic particle according to claim 1, wherein the polyamine compound is at least one selected from the group consisting of polyethyleneimine and polylysine.

3. The cellulosic particle according to claim 1 or 2, wherein the wax is carnauba wax.

4. The cellulosic particle according to any one of claims 1 to 3, wherein the second coating layer further contains a polyvalent metal salt.

5. The cellulosic particle according to any one of claims 1 to 3, wherein the first coating layer contains at least one selected from the group consisting of the polyamine compound and the arginine compound, and the second coating layer contains at least one selected from the group consisting of the linear-chain fatty acid, the linear-chain fatty acid metallic salt, and the amino acid compound.

6. The cellulosic particle according to claim 5, wherein the second coating layer further contains a polyvalent metal salt.

7. The cellulosic particle according to any one of claims 1 to 6, further comprising at least one external additive selected from the group consisting of a silicon-containing compound particle and a metallic soap particle.

8. The cellulosic particle according to claim 7, wherein the silicon-containing compound particle is a silica particle.

9. The cellulosic particle according to any one of claims 1 to 8, wherein a volume-average diameter of the cellulosic particles is 3 $\mu$m or more and less than 10 $\mu$m as determined by the methods disclosed in the description.

10. The cellulosic particle according to any one of claims 1 to 9, wherein an upper geometric standard deviation by number GSDv of the cellulosic particles, as determined by the methods disclosed in the description, is 1.0 or greater and 1.7 or less.

11. The cellulosic particle according to any one of claims 1 to 8, wherein sphericity of the cellulosic particle, as determined by the methods disclosed in the description, is 0.9 or greater.

12. The cellulosic particle according to any one of claims 1 to 11, wherein a number-average molecular weight of the cellulose, as determined by the methods disclosed in the description, is 37000 or more.

13. The cellulosic particle according to any one of claims 1 to 12, wherein surface smoothness of the cellulosic particle, as determined by the methods disclosed in the description, is 80% or higher.

**Patentansprüche**

1. Zellulosepartikel, umfassend:

ein Kernpartikel, das Zellulose als einen Basisbestandteil enthält; und
eine Beschichtungsschicht, die das Kernpartikel abdeckt und aufweist:

eine erste Beschichtungsschicht, die das Kernpartikel abdeckt, und
eine zweite Beschichtungsschicht, die die erste Beschichtungsschicht abdeckt,
wobei prozentuale biologische Abbaurate des Zellulosepartikels für 5 Tage oder 60 Tage, gemessen gemäß JIS K6950:2000 gemessen, entsprechend niedriger als 20 % und 60 % oder höher sind,
wobei das Kernpartikel aus Zellulosepartikel-Vorläufern produziert wird, die aus einer Emulsion einer Celluloseacylatlösung in Ethylacetat und einer
Carboxymethylcellulose-Dispersion in Methyläthylketon, die auf 80 °C erwärmt wurde, mit Natriumhydroxid und dann mit Salzsäure behandelt wurde und dann verseift wurde, hergestellt werden,
wobei die erste Beschichtungsschicht und die zweite Beschichtungsschicht enthalten:

entsprechend eine Polyaminverbindung und ein Wachs, oder
entsprechend eine Fettsäure mit linearer Kette und ein Wachs, oder
entsprechend eine Polyaminverbindung und eine Fettsäure mit linearer Kette, oder
entsprechend eine Polyaminverbindung und eine Aminosäureverbindung, oder entsprechend eine Argininverbindung und ein Metallseifensalz von Fettsäure mit linearer Kette, oder
entsprechend eine Argininverbindung und eine Fettsäure mit linearer Kette, oder
entsprechend eine Argininverbindung und eine Aminosäureverbindung, oder
entsprechend eine Argininverbindung und ein Wachs, oder
entsprechend eine Polyaminverbindung und ein Metallseifensalz von Fettsäure mit linearer Kette,
wobei die Polyaminverbindung ein Polyalkylenimin, Polyallylamin, Polyvinylamin oder Polylysin ist,
die Fettsäure mit linearer Kette Behensäure, Arachinsäure oder Palmitinsäure ist,
die Argininverbindung L-Arginin, D-Arginin, 2-Amino-3-methyl-5-guanidinopentansäure, 2-Amino-3-ethyl-5-guanidinopentansäure oder 2-Amino-3,3-dimethyl-5-guanidinopentansäure ist,
das Wachs Rizinusöl, Paulowniaöl, Leinöl, Fett, Maisöl, Sojaöl, Sesamöl, Rapsöl, Sonnenblumenöl, Reiskleieöl, Kamelienöl, Kokosnussöl, Palmöl, Walnussöl, Olivenöl, Erdnussöl, Mandelöl, Jojobaöl, Kakaobutter, Shea-Butter, Neemöl, Distelöl, Japanwachs, Candelillawachs, Reiskleienwachs, Carnaubawachs, Rosa damascena Blumenwachs, Erdölwachse, synthetische Kohlenwasserstoffwachse, Malinsäure, Glutarsäure, Adipinsäure, Azelainsäure, Sebacinsäure, Dodecandisäure oder C10 bis C25-Alkohole sind,
die Aminosäureverbindung Lauryl-Leucin, Lauryl-Arginin oder Myristyl-Leucin ist,
das Metallsalz von Fettsäure mit linearer Kette das Metallsalz von Fettsäuren mit C10 bis C25 ist, das Metallsalz von Stearinsäure, Palmitinsäure, Laurinsäure, Ölsäure, Linolsäure und Ricinölsäure ist und Metalle in Metallsalzen von Fettsäure mit linearer Kette Magnesium, Calcium, Aluminium, Barium oder Zink umfasst.

2. Zellulosepartikel nach Anspruch 1, wobei die Polyaminverbindung mindestens eine ist, die aus der Gruppe, die aus Polyethylenimin und Polylysin besteht, ausgewählt wird.

3. Zellulosepartikel nach Anspruch 1 oder 2, wobei das Wachs Carnaubawachs ist.

4. Zellulosepartikel nach einem der Ansprüche 1 bis 3, wobei die zweite Beschichtungsschicht ferner ein polyvalentes Metallsalz enthält.

5. Zellulosepartikel nach einem der Ansprüche 1 bis 3, wobei die erste Beschichtungsschicht mindestens eine enthält, das aus der Gruppe, die aus der Polyaminverbindung und der Argininverbindung besteht, ausgewählt wird, und die zweite Beschichtungsschicht mindestens eine enthält, das aus der Gruppe, die aus der Fettsäure mit linearer Kette, dem Metallsalz von Fettsäure mit linearer Kette und der Aminosäureverbindung besteht, ausgewählt wird.

6. Zellulosepartikel nach Anspruch 5, wobei die zweite Beschichtungsschicht ferner ein polyvalentes Metallsalz enthält.

7. Zellulosepartikel nach einem der Ansprüche 1 bis 6, ferner umfassend mindestens ein externes Additiv, das aus der Gruppe, die aus einem Silicium enthaltenden Verbindungspartikel und einem Metallseifenpartikel besteht, ausgewählt wird.

8. Zellulosepartikel nach Anspruch 7, wobei das Silicium enthaltende Verbindungspartikel ein Siliciumdioxidpartikel ist.

9. Zellulosepartikel nach einem der Ansprüche 1 bis 8, wobei ein volumenmittlerer Durchmesser der Zellulosepartikel 3 $\mu$m oder mehr und weniger als 10 $\mu$m beträgt, wie durch die in der Beschreibung offenbarten Verfahren bestimmt.

10. Zellulosepartikel nach einem der Ansprüche 1 bis 9, wobei eine obere geometrische Standardabweichung GSDv nach Anzahl der Zellulosepartikel, wie durch die in der Beschreibung offenbarten Verfahren bestimmt, 1,0 oder größer und 1,7 oder kleiner ist.

11. Zellulosepartikel nach einem der Ansprüche 1 bis 8, wobei Sphärizität des Zellulosepartikels, wie durch die in der Beschreibung offenbarten Verfahren bestimmt, 0,9 oder größer ist.

12. Zellulosepartikel nach einem der Ansprüche 1 bis 11, wobei ein zahlenmittleres Molekulargewicht der Zellulose, wie durch die in der Beschreibung offenbarten Verfahren bestimmt, 37.000 oder mehr beträgt.

13. Zellulosepartikel nach einem der Ansprüche 1 bis 12, wobei Oberflächenglätte des Zellulosepartikels, wie durch die in der Beschreibung offenbarten Verfahren bestimmt, 80 % oder höher ist.

**Revendications**

1. Particule cellulosique comprenant :

une particule centrale contenant de la cellulose en tant que constituant de base ; et
une couche de revêtement recouvrant la particule centrale, ayant :

une première couche de revêtement recouvrant la particule centrale, et
une deuxième couche de revêtement recouvrant la première couche de revêtement,
dans lequel des dégradations biologiques en pourcentage de 5 jours et de 60 jours de la particule cellulosique mesurées conformément à JIS K6950:2000 sont inférieures à 20 % et à 60 % ou plus, respectivement,
dans lequel la particule centrale est produite à partir de précurseurs de particules de cellulose qui sont préparés à partir d'une émulsion d'une solution d'acylate de cellulose dans l'acétate d'éthyle et d'une dispersion de cellulose carboxyméthyl dans le méthyléthylcétone qui a été chauffée à 80 °C avec de l'hydroxyde de sodium, puis traitée avec de l'acide chlorhydrique, puis saponifiée,
dans lequel la première couche de revêtement et la deuxième couche de revêtement contiennent

un composé de polyamine et une cire, respectivement, ou
un acide gras en chaîne linéaire et une cire, respectivement, ou
un composé de polyamine et un acide gras en chaîne linéaire, respectivement, ou
un composé de polyamine et un composé d'acide aminé, respectivement, ou
un composé d'arginine et un sel métallique d'acide gras en chaîne linéaire, respectivement, ou
un composé d'arginine et un acide gras en chaîne linéaire, respectivement, ou
un composé d'arginine et un composé d'acide aminé, respectivement, ou
un composé d'arginine et une cire, respectivement, ou
un composé de polyamine et un sel métallique d'acide gras en chaîne linéaire, respectivement,
dans lequel le composé de polyamine est une polyalkylèneimine, une polyallylamine, une polyvinylamine ou une polylysine,
l'acide gras en chaîne linéaire est l'acide béhénique, l'acide arachidique ou l'acide palmitique,
le composé d'arginine est L-arginine, D-arginine, acide 2-amino-3-méthyl-5-guanidinopentanoïque, acide 2-amino-3-éthyl-5-guanidinopentanoïque ou acide 2-amino-3,3-diméthyl-5-guanidinopentanoïque,
la cire est de l'huile de ricin, de l'huile de paulownia, de l'huile de lin, de la matière grasse, de l'huile de maïs, de l'huile de soja, de l'huile de sésame, de l'huile de colza, de l'huile de tournesol, de l'huile d'orge, de l'huile de camélia, de l'huile de coco, de l'huile de palme, de l'huile de noix, d'huile d'olive, d'huile d'arachide, d'huile d'amande, d'huile de jojoba, de beurre de cacao, de beurre de karité, d'huile de neem, d'huile de carthame, de cire de Japon, de cire de candelilla, de cire de son de riz, de cire de carnauba, de cire de fleur de Rosa damascena, de cires pétrolières, de cires hydrocarbonées synthétiques, d'acide malique, d'acide glutarique, d'acide adipique, d'acide azélaïque, d'acide sébacique, d'acide dodecanedioïque ou d'alcools C10 à C25,
le composé d'acide aminé est lauryl leucine, lauryl arginine ou myristyl leucine,
le sel métallique d'acide gras en chaîne linéaire est le sel métallique des acides gras C10 à C25 qui est le sel métallique d'acide stéarique, d'acide palmitique, d'acide laurique, d'acide oléique, d'acide linoléique et d'acide ricinoléique, et les métaux dans les sels métalliques d'acides gras en chaîne linéaire incluent du magnésium, du calcium, de l'aluminium, du baryum ou du zinc.

2. Particule cellulosique selon la revendication 1, dans laquelle le composé de polyamine est au moins l'un sélectionné parmi le groupe constitué de polyéthylèneimine et de polylysine.

3. Particule cellulosique selon la revendication 1 ou la revendication 2, dans laquelle la cire est de la cire de carnauba.

4. Particule cellulosique selon l'une quelconque des revendications 1 à 3, dans laquelle la deuxième couche de revêtement contient en outre un sel de métal polyvalent.

5. Particule cellulosique selon l'une quelconque des revendications 1 à 3, dans laquelle la première couche de revêtement contient au moins l'un sélectionné parmi le groupe constitué du composé de polyamine et du composé d'arginine, et la deuxième couche de revêtement contient au moins l'un sélectionné parmi le groupe constitué de l'acide gras en chaîne linéaire, du sel métallique d'acide gras en chaîne linéaire et du composé d'acide aminé.

6. Particule cellulosique selon la revendication 5, dans laquelle la deuxième couche de revêtement contient en outre un sel de métal polyvalent.

7. Particule cellulosique selon l'une quelconque des revendications 1 à 6, comprenant en outre au moins un additif externe sélectionné parmi le groupe constitué d'une particule de composé contenant du silicium et d'une particule de savon métallique.

8. Particule cellulosique selon la revendication 7, dans laquelle la particule de composé contenant du silicium est une particule de silice.

9. Particule cellulosique selon l'une quelconque des revendications 1 à 8, dans laquelle un diamètre moyen en volume des particules cellulosiques est de 3 $\mu$m ou plus et inférieur à 10 $\mu$m, tel que déterminé par les méthodes divulguées dans la description.

10. Particule cellulosique selon l'une quelconque des revendications 1 à 9, dans laquelle un écart-type géométrique supérieur par nombre GSDv des particules cellulosiques, tel que déterminé par les méthodes divulguées dans la description, est de 1,0 ou plus et de 1,7 ou moins.

11. Particule cellulosique selon l'une quelconque des revendications 1 à 8, dans laquelle la sphéricité de la particule cellulosique, tel que déterminé par les méthodes divulguées dans la description, est de 0,9 ou plus.

12. Particule cellulosique selon l'une quelconque des revendications 1 à 11, dans laquelle un poids moléculaire moyen en nombre de la cellulose, tel que déterminé par les méthodes divulguées dans la description, est de 37000 ou plus.

13. Particule cellulosique selon l'une quelconque des revendications 1 à 12, dans laquelle la régularité de surface de la particule cellulosique, tel que déterminé par les méthodes divulguées dans la description, est de 80 % ou plus.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6872068 B
- JP 6855631 B
- WO 2017104587 A2
- WO 2016174503 A1
- WO 2016098910 A1

- EP 0273890 A1
- EP 0421581 A1
- JP 2021021044 A
- JP 2021021045 A
- JP 6921293 B